# EUROPEAN PATENT APPLICATION

(11) **EP 1 153 594 A2**
(43) Date of publication of application: **14.11.2001**
(21) Application number: 01109654.2
(22) Date of filing: 19.04.2001
(51) Int. Cl.: A61K 6/083, A61K 6/02, A61K 7/043, A61K 7/047, A61B 1/24

(54) **Cosmetic coating composition, especially for teeth, remover, and intraoral lip supporter**

(30) Priority: 21.04.2000 JP 2000121835; 14.03.2001 JP 2001072640
(71) Applicant: Coden Co., Ltd., Tokyo 114-0024 (JP)
(72) Inventor: Koda, Yoshiharu, Tokyo 114-0024 (JP); Tsukagoshi, Ken, Katsuura-shi, Chiba 299-5235 (JP)
(74) Representative: Fuchs Mehler Weiss & Fritzsche

(57) **Abstract**

A cosmetic coating composition comprising acrylic acid type copolymers, pyrrolidone type copolymers, and an alcohol solvent, a remover for dental coating composition comprising an alcohol solvent and a gelling agent and containing said alcohol solvent in an amount of not less than 20 mass % based on the total mass of the remover, and a intraoral lip supporter for fixing lips to gums. Further, a method for applying a coating composition characterized by applying a coating composition containing a white powdered pigment as a primer coating, and then overcoating a coating composition containing a chromatic color type pigment or microfine leaves, and a method for applying a coating composition with using said supporter.

## Description

### Background of the Invention

### Field of the Invention:

This invention relates to a cosmetic coating composition. More particularly, this invention relates to a cosmetic coating composition for exalting the quality of fashion of the skin, nails, or teeth by whitening, coloring, or lustering the surface thereof.

Further, this invention relates to a remover for removing the applied layer of a coating composition and an intraoral lip supporter to be used in applying a coating composition to the surface and drying the applied layer of the coating composition.

### Description of Related Art:

Heretofore, in the dental field, the coating composition is used on teeth as a part of therapy. Such coating of teeth is aimed at enabling the surface of teeth or artificial teeth stained by disease or therapy to approximate closely to the color of natural teeth or preventing dental caries, periodontal disease, bacterial plaque, etc. by dint of an effective component such as an antibacterial agent or a fluorine preparation added in advance in the coating composition. On the other hand, in recent years, the development of a coating composition intended to exalt the beauty of teeth has also been advancing. Specifically, the beautification of teeth is accomplished by applying to teeth a coating composition having such a pigment as titanium dioxide or mica titanium mixed with a polymer solution thereby making the teeth look white. The coating of teeth performed in this manner is aimed at temporarily whitening the teeth stained with tobacco tar or tea incrustations or exalting the beautiful appearance of a face with whiteness or luster.

As a film-forming component of the coating composition, varying acrylic acid type polymers are used (refer to JP-A-11-92,350). It has been known to use a coating composition having polyvinyl pyrrolidone in an acrylic acid type polymer (JP-A-06-256,147) and a coating composition having vinyl acetate incorporated likewise (JP-A-09-100,215 and JP-A-09-151,123) for the purpose of improving polymers in solubility, dispersion stability, gloss, applicability, and fastness of adhesion.

These conventional products, however, have been at a disadvantage in entailing great inconvenience of use as because of seriously uneven application of a composition to a surface and very slow drying of an applied layer, incurring easy peeling due to weak adhesive force to teeth, retaining the effect barely for several hours, failing to realize the expected impartation of whiteness after coating, and inducing dispersion of a composition only with unduly low stability.

Further, the conventional products have been aimed at approximating the color of a coating film closely to the color of natural teeth or directing the luster or gloss imparted by the coating film to emphasize the whiteness of teeth. None of them has pursued such quality of fashion as treasures the vivid tinge and gloss.

Of course, the problems of uneven application, slow drying, and defective performance in adhesion, film-forming property, and dispersion stability mentioned above are unsettled tasks which are shared with a coating compositions for nail.

The lamé (hereinafter referred to occasionally as "microfine leaves") is a decorative additive which is used in such cosmetic articles as nail manicure. A great variety of lamé-containing compositions have been developed to date. Since the lamé has a large specific gravity, however, it does not permit easy dispersion and entails uneven coating seriously. It is further at a disadvantage in easily peeling from an applied layer of a composition.

Further, such cosmetic articles as lamé-containing foundations and creams have been developed recently. In these cosmetic articles, the lamé manifests low adhesiveness and tends to peel with elapse of time or on exposure to perspiration. The lamé used in most of such cosmetic articles has a microfine size. The lamé of a large size is not used in these cosmetic articles because it is liable to peel.

Incidentally, the coating composition of this sort, when necessary, must facilitate easy removal. In this respect, the conventional products are removed from teeth, for example, by being directly wiped with a wad or a cloth impregnated with a solvent. The wiping, however, is incapable of completely removing the coating composition adhering to grooves or gaps between adjacent teeth. Further, the use of the coating composition entails the displeasure ascribable to the smell of the solvent and the pungency inflicted on the mucous membrane.

Moreover, in the actual application of such a coating composition to teeth, the upper and lower lips must be fixed so as to be prevented from touching the teeth. The retention of the lips in this posture between the time of application and the time of thorough drying, however, entails considerable difficulty.

### Summary of the Invention

This invention has been produced in view of the problems mentioned above and has for a primary object thereof the provision of a cosmetic coating composition which dries quickly after application to the skin, nails, or teeth, manifests appropriate adhesive force, possesses fine luster, and excels in dispersion stability.

The second object of this invention is to provide a dental coating composition which is capable of coloring teeth and imparting a beautiful gloss thereto.

The third object of this invention is to provide a cosmetic coating composition which produces lasting satisfactory dispersion of microfine leaves such as lamé and possesses a satisfactory adhesive property.

The fourth object of this invention is to provide a remover which is capable of quickly and easily removing dental coating compositions from teeth.

Further, the fifth object of this invention is to provide a supporter which, by fixing the upper and lower lips in a state opened on the gums' side so as to avoid touching the teeth during the application of a dental coating composition to the teech, aids in the work of application and the work of drying the applied layer of the composition and further to provide a method for the application of the coating composition of this invention by the use of the supporter.

Therefore, the objects of this invention mentioned above will be accomplished by the following items (1)-(10).
(1) a cosmetic coating comprising
   at least one polymer I which is a copolymer of at least two monomers selected from the group consisting of monomers represented by the chemical formula 1: 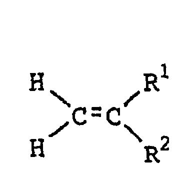 wherein, R¹ represents a hydrogen atom or a methyl group, R² represents -COOR³ or -CONHR³, wherein R³ represents a hydrogen atom or a linear, branched, or cyclic alkyl group of 1-10 carbon atoms,
   at least one polymer II which is a copolymer of at least two monomers selected from the group consisting of monomers represented by the chemical formula 2: 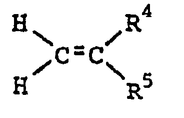 wherein, R⁴ represents a hydrogen atom or a methyl group, R⁵ represents -COOR⁶, an acetoxyl group, or a 2-pyrrolidyl group, wherein R⁶ represents a linear, branched, or cyclic alkyl group of 1-6 carbon atoms, providing that in at least one of said at least two monomers, R⁶ represents a 2-pyrrolidyl group), and
   an alcohol solvent.
(2) Said composition, which further comprises a powdered pigment, wherein the powdered pigment is a chromatic color type pigment, and characterized by applying on teeth.
(3) Said composition, which further comprises microfine leaves.
(4) A dental coating composition comprising at least one polymer selected from the group consisting of acrylic acid type copolymers, vinyl acetate type copolymers, and 5 pyrrolidone type copolymers, an alcohol solvent, and a chromatic color type pigment.
(5) A cosmetic coating composition comprising at least one polymer selected from the group consisting of acrylic acid type copolymers, vinyl acetate type copolymers, and pyrrolidone type copolymers, an alcohol solvent, ethyl cellulose, and microfine leaves.
(6) A remover for a dental coating composition, characterized by comprising an alcohol solvent and a gelling agent and containing said alcohol solvent in an amount of not less than 20 mass % based on the total mass of the remover.
(7) An intraoral lip supporter for use in applying a dental coating composition and drying the applied dental coating composition, characterized by forming a plastic material possessing viscosity at least during the time of use and serving the purpose of wedging itself between gums and lips thereby opening the lips, and fixing the lips to the gums with the teeth kept meanwhile in an exposed state.
(8) An intraoral lip supporter for use in applying a dental coating composition and drying the applied dental coating composition, characterized by possessing a construction curved in conformity with the shape of a gum and serving the purpose of wedging itself between the gums and the upper and lower lips thereby fixing the lips to the gums with the teeth kept meanwhile in an exposed state.
(9) A method for applying a coating composition to nail or tooth, characterized by applying said composition containing a white powdered pigment to the nail or tooth as a primer coating, and then overcoating said composition containing a chromatic color type pigment or microfine leaves.
(10) A method for applying said composition to a tooth by the use of said supporter.

The dental coating composition of this invention is enabled by using the polymer I which is an acrylic acid type copolymer and the polymer II which is a pyrrolidone type copolymer in combination to acquire improvement in quick-drying property, luster, lasting dispersion stability, and durability of use as compared with the conventional composition having polyvinyl pyrrolidone or polyvinyl acetate added solely thereto. Specifically, it can form a highly satisfactory coating film on the skin, nails, or teeth. When it is applied to teeth and then brushed with an ordinary dentifrice containing an ordinary abrasive, it is not peeled away from the teeth. Thus, it allows the user to enjoy beautification of his teeth without changing his daily habit such as toothbrushing.

Further, owing to the mixing of ethyl cellulose therein, the composition can be dispersed and retained in the dispersed state very stably for a long time without entailing either precipitation of pigment and microfine leaves which are liable to precipitate or separation of a liquid layer. The coating composition of this quality will not induce any increase of viscosity or any decline of quick-drying property in consequence of the addition to the dispersion stability. In the aspect of beauty, the coating composition of this invention is capable of producing excellent coloration and realizing variegated metallic brilliance emanating from microfine leaves.

The chromatic color type coating composition of this invention which contains a chromatic color pigment is intended to realize beautification of teeth based on a novel concept and is capable of beautifully coloring teeth.

The coating composition of this invention which contains microfine leaves is enabled by the mixing therein of ethyl cellulose to be dispersed and retained in the dispersed state very stably for a long time without entailing either precipitation of microfine leaves which are liable to precipitate or separation of a liquid layer. It also excels in adhesiveness of microfine leaves.

The white color type coating composition, when used as a primer, subdues the irregularities and scratches on the surface of nails or teeth and enables a chromatic color type coating composition or a coating composition containing microfine leaves to produce beautiful coloration as an overcoat. Thus, it realizes the surface of an applied film possessing greater smoothness than when the respective compositions are applied independently.

The remover of this invention, owing to its high alcohol content, can easily remove the coating film from the teeth by an ordinary brushing work. Since it possesses viscosity proper for brushing, it is allowed to acquire improved workability without entailing the phenomenon of weeping on the gums.

The supporter of this invention can fix the lips to the gums with proper viscous force without inflicting displeasure or pain upon the user. Since it exceptionally excels in the ability to absorb water, it immediately acquires increased viscosity and fixes the lips after it has been set in position in the oral cavity. Thus, it can retain its viscous force for a duration enough for the work of application and the work of drying.

Accordingly, by the cosmetic coating composition, remover, intraoral lip supporter, and method of application of this invention, the beautification of the skin, nails, or teeth which excels in workability and physical properties and realizes beautiful appearance can be enjoyed as a daily pleasure.

### Brief Description of the Drawing

Fig. 1 is a perspective view illustrating one example of the state which is assumed by a supporter of this invention prior to use.

Fig. 2 is a cross section illustrating the state of the interior of an oral cavity assumed when the upper and lower lips are vertically opened to expose the gums prior to the attachment of the supporter of this invention.

Fig. 3 is a cross section illustrating the state of the interior of an oral cavity assumed when the supporter of this invention is set in place.

Fig. 4 is a diagram illustrating the external appearance of a mouth assumed when the supporter of this invention is set in place.

Fig. 5 is a diagram illustrating with a broken line the supporter in addition to the diagram of the external appearance of Fig. 4 to facilitate comprehension of the state of attachment of the supporter of this invention.

Fig. 6 is a front view of an opened lips supporting member illustrating a mode of embodiment of the supporter of this invention; A representing an annular shape, B a shape opened at one part, C a shape opened at two portions, and D a partly modified shape.

Fig. 7 is a plan view E and a front view F of an opened lips supporting member illustrating one mode of embodiment of the supporter of this invention.

Fig. 8 is cross sections I-K illustrating one mode of embodiment of a connecting member to be used in the supporter of this invention.

Fig. 9 is a front view L illustrating one mode of embodiment of the supporter of this invention and a perspective view M of a shock absorbing member.

Fig. 10 is a perspective view illustrating one mode of embodiment of the shape of the supporter of this invention prior to use.

Fig. 11 is a cross section illustrating the state of the interior of an oral cavity assumed when the supporter of this invention is mounted in place.

### Description of the Preferred Embodiments

Now, this invention will be described in detail below.

The cosmetic coating composition of this invention is characterized by comprising;
at least one polymer I which is a copolymer of at least two monomers selected from the group consisting of monomers represented by the chemical formula 1: 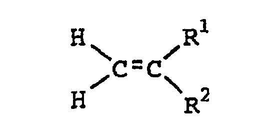 wherein, R¹ represents a hydrogen atom or a methyl group, R² represents -COOR³ or -CONHR³, wherein R³ represents a hydrogen atom or a linear, branched, or cyclic alkyl group of 1-10 carbon atoms,
at least one polymer II which is a copolymer of at least two monomers selected from the group consisting of monomers represented by the chemical formula 2: 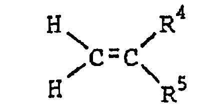 wherein, R⁴ represents a hydrogen atom or a methyl group, R⁵ represents -COOR⁶, an acetoxyl group, or a 2 -pyrrolidyl group, wherein R⁶ represents a linear, branched, or cyclic alkyl group of 1-6 carbon atoms, providing that in at least one of the at least two monomers, R⁶ represents a 2-pyrrolidyl group, and an alcohol solvent.

First, the polymer I will be explained. The polymer I is an acrylic acid-ester copolymer and is used for realizing excellent gloss and durability after drying.

In the monomer which forms the polymer I and is represented by the chemical formula 1, R² represents a hydrogen atom, -COOR³, or -CONHR³, wherein R³ represents a hydrogen atom or a linear, branched, or cyclic alkyl group of 1-10 carbon atoms. Concrete examples of such alkyl group include, but not limited to, methyl, ethyl, propyl, isopropyl, n-butyl, t-butyl, sec-butyl, pentyl, isopentyl, cyclopentyl, hexyl, isohexyl, cyclohexyl, heptyl, isoheptyl, cycloheptyl, octyl, isooctyl, cyclooctyl, nonyl, isononyl, cyclononyl, decyl, isodecyl, and cyclodecyl.

Preferably, the polymer I is a copolymer of monomers selected from among (meth) acrylic acid, methyl (meth) acrylate, ethyl (meth)acrylate, isopropyl (meth)acrylate, n-butyl (meth)acrylate, t-butyl (meth)acrylate, isobutyl (meth)acrylate, and N-octylacrylic acid amide.

One embodiment of the polymer I is a copolymer formed of at least two monomers selected from the group consisting of alkyl (meth)acrylates and (meth)acrylic acid. Specifically, a t-butyl acrylate-ethyl acrylate-methacrylic acid copolymer is particularly excellent in adhesiveness, film-forming property, durability, and luster.

Another embodiment of the polymer I is a copolymer which contains N-octyl-acrylic acid amide as the monomer of the chemical formula 1 mentioned above. Specifically, an isobutyl methacrylate-N-octylacrylic acid amide-acrylic acid copolymer is advantageously used for the reason which will be more specifically described herein below.

The content of the polymer I in the composition is in the range of 0.1-50 mass %, preferably 2-30 mass %, based on the total mass of the composition. If this content falls smaller than 0.1 mass %, the film-forming property of the composition is degraded. Conversely, if the content exceeds 50 mass %, the solubility and dispersibility of the composition in a solvent are degraded, thereby to render difficult the formation of a uniform film and form a cause for uneven application of the composition.

Now, the polymer II will be explained below. The polymer II is a pyrrolidone typecopolymer which never fails to contain a 2-pyrrolidyl group of the following formula in the construction thereof.

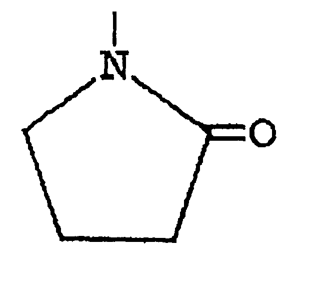

In the monomer forming the polymer II and represented by the chemical formula 2, R⁴ represents a hydrogen atom or a methyl group, R⁵ a -COOR⁶, an acetoxyl group, or a 2-pyrrolidyl group, wherein R⁶ a linear, branched, or cyclic alkyl group of 1-6 carbon atoms. Concrete examples of the substituent R⁵ include, but not limited to, methyl, ethyl, propyl, isopropy, t-butyl,n-butyl,sec-butyl,pentyl,isopentyl,cyclopentyl, hexyl, isohexyl, and cyclohexyl.

One embodiment of the polymer II is a copolymer of monomers selected from among (meth) acrylic acid, methyl (meth)acrylate, ethyl (meth)acrylate, isopropyl (meth)acrylate, n-butyl (meth)acrylate, t-butyl (meth)acrylate, isobuyl (meth)acrylate, vinyl acetate, and N-vinyl-2-pyrrolidone, providing that at least one monomer is N-vinyl-2-pyrrolidone. Specifically, a N-vinyl-2-pyrrolidine-vinyl acetate copolymer and N-vinyl-2-pyrrolidone-vinyl acetate type copolymer are used advantageously.

The content of the polymer II in the composition is in the range of 1-70 mass %, preferably 5-30 mass %, based on the total mass of the composition. If this content falls smaller than 1 mass %, the shortage will be at a disadvantage in preventing the effect of addition of the polymer II from being manifested. Conversely, if the content exceeds 70 mass %, the excess will be at a disadvantage in degrading the dispersibility of the composition in a solvent, rendering formation of a uniform film difficult, consequently entailing uneven application of the composition, and failing to produce an effect commensurate with the excess of the amount added.

By using the polymer described above, the composition is enabled to retain appropriate adhesiveness to the surface of the skin, nails, and teeth. The composition, when applied to teeth, for example, is enabled to form a coating film which manifests excellent adhesiveness and durability, incurs no easy peeling by friction as with food and drink, and, when necessary, allows easy removal with a remover. By using the polymer I in combination with the polymer II, it is made possible to improve the film-forming property, quick drying property, dispersion stability, and luster of the composition more conspicuously than using such a homopolymer as polyvinyl pyrrolidone or polyvinyl acetate. Further, the composition allows mixing therein of a polymer other than the polymers I and II or a resin such as, for example, shellac.

Then, the alcohol solvent will be explained below. The alcohol solvent does not need to be particularly limited. A lower alcohol solvent such as ethanol is preferably used as the alcohol solvent because it avoids emitting a pungent odor and is obtained easily. The content of the alcohol solvent in the composition is in the range of 25-98.9 mass %, preferably 30-95 mass %, based on the total mass of the composition. If this content falls smaller than 25 mass %, the shortage will be at a disadvantage in rendering dissolution of a polymer difficult. Conversely, if the content exceeds 98.9 mass %, the excess will be at a disadvantage in excessively lowering the content of the polymer and consequently compelling an applied film to suffer from deficiency in gloss and film-forming property.

Now, the coating composition containing a powdered pigment (white type) will be explained below. The powdered pigment is added to the composition for the purpose of making nails or teeth coated with the composition look white. Concrete examples of the powdered pigment include, but not limited to, titanium dioxide, mica titanium, silicon dioxide, talc, kaolin, aluminum oxide, titanium oxide sol, zinc oxide, low-temperature sintered zinc oxide, silicic anhydride, and sericite. These compounds have such advantages as manifesting good dispersibility in a solvent and possessing stability and cheepness. When the surface of teeth coated with the composition is exposed to light, the applied film can realize a beautiful pearly color because of the irregular reflection of the particles of powdered pigment. Besides the powdered pigments enumerated above, such pearl essences as powdered fish scale and powdered shell are likewise usable.

Then, the dental coating composition containing a chromatic color type pigment will be explained below. The pigment possessing a chromatic color does not need to be particularly limited. It may be a natural or synthetic pigment or dye material. The concrete examples of the chromatic color type pigment are as follows.

Red color: Carmine-coated mica titanium, red color No. 226-coated mica titanium, black iron oxide/Carmine-coated titanium oxide, iron oxide red-coated mica, iron oxide/titanium oxide sinter, and iron oxide red.

Blue color: Black iron oxide/Prussian blue-coated mica titanium, black iron oxide-coated mica titanium, Prussian blue-coated mica, Prussian blue-coated mica titanium, ultramarine blue, and Prussian blue.

Green color: Black iron oxide-coated mica titanium, chromium oxide, black iron oxide-coated mica titanium, chromium oxide-coated mica titanium, iron oxide red/black iron oxide/Prussian blue-coated mica titanium, iron oxide red/Prussian blue-coated mica titanium, cobalt titanate, lithium cobalt titanate, and sodium copper chlorophyllin.

Purple color: Carmine/Prussian blue-coated mica titanium, ultramarine blue violet, and ultramarine blue pink.

Golden color: Iron oxide red/black iron oxide-coated mica titanium, iron oxide red/Prussian blue-coated mica titanium, and iron oxide red-coated mica titanium.

Silver color: Mica titanium, black iron oxide-coated mica titanium, bismuth oxychloride, and bismuth oxychloride-coated mica.

Orange color: iron oxide red-coated mica titanium and iron sulfide.

Yellow color: Yellow iron oxide-coated mica and yellow iron oxide/calcium carbonated-coated mica.

Further, numerous neutral colors can be realized by mixing various pigments and dye materials mentioned above. More beautiful coloration can be realized by further mixing such white pigment as titanium dioxide in a proper amount. As a coloring material other than those enumerated above, the cosmetic tar pigment (a statutory pigment) is also usable. By applying such a chromatic color coating composition as described above to teeth, it is made possible to realize impartation of a varying color to the surface of the teeth.

The content of the powdered pigment in the composition is preferably in the range of 0.1-50 mass %, more preferably 1-20 mass %, based on the total mass of the composition. If this content falls smaller than 0.1 mass %, the shortage will be at a disadvantage in bringing an unduly low effect of the addition of the powdered pigment. Conversely, if the content exceeds 50 mass %, the excess will induce uneven application of the composition.

The average particle diameter of the powdered pigment is preferably in the range of 0.01-250*µ*m, more preferably 0.04-30*µ*m. If the particle diameter falls smaller than 0.01 *µ*m, the shortage will be at a disadvantage in rendering preparation of the powdered pigment difficult. Conversely, if the particle diameter exceeds 250 *µ*m, the excess will be at a disadvantage in degrading coloration of the composition, rendering preparation of a uniformly dispersed solution of the composition difficult, and inducing uneven application of the composition.

Now, the coating composition containing microfine leaves will be explained below. The term "microfine leaves" as used herein means lamé, microfine foils, or microfine films. Concrete examples of the microfine leaves include, but not limited to, metal foils, film products such as a polyethylene terephthalate/aluminum/epoxy laminate film and a polyethylene terephthalate/metal laminate film which are obtained by coating a metal foil with a thin film of a varying color, and products obtained by finely cutting a polyethylene terephthalate/polymethyl methacrylate laminated film and a polyethylene terephthalate/polyolefin laminated film. The gloss and the color of such microfine leaves are varied with the shape, thickness, and size of the leaves. These species of microfine leaves may be in general metallic colors such as golden color, silver color, and copper color and such metallic colors as are produced by superposing chromatic colors. They may be transparent or opaque. They may be those of a single color or those of such a color as is produced by mixing two or more colors. By adding such microfine leaves to the coating composition, it is made possible to form a beautiful coating film which emits a vivid metallic gloss or the gloss of a rainbow color on exposure to a light.

The coating composition of this invention is allowed to mix a dispersant therein for the purpose of attaining stable dispersion of such particles of inferior dispersibility as the lamé, i.e. microfine foils. Concrete examples of the dispersant include, but not limited to, such cellulose derivatives as ethyl cellulose, hydroxyethyl cellulose, hydroxypropyl cellulose, and sodium carboxymethyl cellulose, such macromolecular polymers as (meth)acrylic acid type copolymers, N-vinyl-2-pyrrolidone type copolymers, polyvinyl pyrrolidone, polyvinyl acetate, and vinyl acetate type copolymers, such polysaccharides as sodium alginate, dextran sodium sulfate, chondroitin sodium sulfate, pectins, carrageenin, galactomannan, and gum arabic, viscosity enhancing silica, montmorillonite, and bentonite may be cited. Ethyl cellulose is advantageous as compared with the other cellulose derivative type dispersants because it retains microfine leaves or a pigment in a fully sufficiently dispersed state for a long time and entails substantially no formation of precipitate.

In the cosmetic coating composition of this invention, the pigment 0.01-250 m in average particle diameter and the microfine leaves less than 0.4 mm in width are dispersed with fully satisfactory stability. Such microfine leaves as Daiyahologram HG-S40EP in a rainbow color (made by Daiya Kogyo Co.) and DC Glitter Gold 1 #0.1 in a golden color (made by Diachemco Co.) which have large specific gravities and such microfine leaves as measure not less then 0.4 mm in width, however, have the possibility of entailing degradation of the dispersion stability in a composition and easily peeling while in use in a composition. Further addition of the alkyl (meth)acrylate-(meth)acrylic acid copolymer mentioned above as the polymer I, therefore, enables the microfine leaves of such a relatively large size as mentioned above to be dispersed stably for a long time and reinforced in resistance to peeling. The copolymer does not need to be limited to what has been just mentioned. As concrete examples of the copolymer, Leoarl MS-100 and Leoarl MS-200(made by Lion Co.), Daitosol 5000AD(made by Daitokasei Co.), Yodosol GH28 and Yodosol GH800(made by Nihon NSC Co.), and Luvimer 36D, Luvimer Low VOC, and Luviflex Soft(made by BSF Japan Co.).

Further, the cosmetic coating composition of this invention, by further adding therein a copolymer containing N-octylacrylic acid amide as a monomer mentioned above, is enabled to impart to the microfine leaves having a large specific gravity or measuring not less than 0.4 mm in width improved resistance to peeling.

Now, the method for synthesizing the polymers I and II of this invention will be explained below. The polymers of this invention are synthesized not by any particularly discriminated method but by any of the methods universally known in the trade. One example will be cited below.

First, a flask is charged with monomers destined to form a target copolymer. The monomers in the flask are bubbled with an inert gas (such as, for example argon gas) and heated to a properly elevated temperature. The hot mixture of monomers consequently formed and a polymerization initiator (such as, for example, azobisisobutyronitrile or benzoyl perchloride or peroxide) added thereto dropwise are left reacting at a proper temperature for several hours. The result of this reaction is washed and refined several times with water and a solvent (such as ethanol) and then dried (by the use of hot air or magnesium sulfate, etc.) to obtain the target copolymer. The copolymer thus obtained may be mixed into a solution by being disolved in such a solvent as ethanol which is usable in the coating composition.

Now, the other components which are added in the coating composition of this invention will be explained below. First, the composition mentioned above may contain a surfactant therein for the purpose of improving the dispersion stablitiy of the polymers and the pigment. The surfactant may be any of the surfactants which are generally used in coating compositions. Concrete examples of the surfactant include, but not limited to, alkyl phosphates such as lauryl sodium sulfate and lauryl sodium phosphate, acylamino acid salts such as acyl sodium glutamate and acyl sodium sarcosinate, sorbitan fatty acid esters such as sorbitan trioleate and polyoxyethylene sorbitan fatty acid esters, sucrose fatty acid esters, polyoxyethylene fatty acid esters, and castor oil type surfactants such as polyoxyethylene castor oil and polyoxyethylene cured castor oil. The content of the surfactant in the composition is properly in the range of 0.1-20 mass %, preferably in the range of 1-10 mass %. If the content of the surfactant falls smaller than 0.1 mass %, the shortage will be at a disadvantage in lowering the effect of addition of the surfactant. Conversely, if this content exceeds 20 mass %, the excess will be at a disadvantage in unduly heightening the viscosity of the composition.

In addition to the components mentioned above, bactericidal agents such as benzalkonium chloride and chlorhexidine chloride, caries preventing agents and dentin reinforcing agents such as sodium monofluorophosphate and sodium fluoride, enzymes such as dextranase, edulcorants as sodium saccharate and sorbit, antiseptic agents such as benzoic acid, p-hydroxybenzoic acid, and paraben, flavoring agent such as menthol, spearmint, and peppermint, and stabilizing agents may be added to the composition.

Now, the method for producing the dental coating agent of this invention will be explained below. The polymer I, polymer II, powdered pigment, surfactant, etc. which have been weighed out in respectively prescribed amounts are added to the alcohol solvent and mixed together by the use of a stirrer or an agitating device. By using a homomixer (the product of SMT Co. or the product of Mizuho Kogyo Co.) in the work of mixing and stirring mentioned above, it is made possible to obtain a coating composition which profoundly excels stability in dispersion. For the purpose of obtaining a coating composition possessing excellent stability in dispersion, it is preferable to operate the homomixer at a rotational frequency of about 5000 rpm per 100g of a composition for a duration in the range of 1-20 minutes, with the kind of a pigment and the contents of polymers suitably adjusted. A coating composition of a varying quality can be produced by adding varying kinds of pigment and lamé to the coating composition prepared as described above.

The viscosity of the coating composition of this invention at 20°C is properly in the range of 0.005-10 Pa · s, preferably 50-400 mPa · s, and more preferably 150-200 mPa · s. If the viscosity falls smaller than 0.005 Pa · s, the shortage will be at a disadvantage in suffering the solution of the coating composition being applied to teeth to lower. Conversely, if the viscosity exceeds 10 Pa · s, the excess will be at a disadvantage in suffering the solution to be applied unevenly to teeth.

The cosmetic coating composition described above can be applied to the skin, nails, or teeth. It may be applied to nails or teeth like ordinary manicure, to lids to form eye lines, or partially to the skin of the face, hands, or feet to deposit microfine leaves thereon.

Now, the chromatic color dental coating composition of this invention will be explained below. The chromatic color dental coating composition comprises a polymer selected from the group consisting of acrylic acid type copolymers, vinyl acetate type copolymers, and pyrrolidone type copolymers, an alcohol solvent, and a chromatic color type pigment. When this composition is applied to teeth, the teeth can be colored in a vivid tint and is enabled to acquire an improved fashioning property.

The acrylic acid type copolymers, vinyl acetate type copolymers, and pyrrolidone type copolymers mentioned above are invariably usable so long as they are capable of forming a film. They may be used either singly or in the form of a mixture of two or more members. To be specific, the dental coating composition can use the same polymers as the polymer I or polymer II which are used for the cosmetic coating composition mentioned above. Preferably, t-butyl acrylate-ethyl acrylate-methacrylic acid copolymers which excel in adhesiveness with nails or teeth, film-forming property, durability, and luster or alkyl acrylates N-octylacrylic acid amide copolymers are preferably used.

The content of the copolymer in the composition is generally in the range of 0.1-50 mass %, preferably 2-30 mass %, based on the total mass of the composition, through depending on the kind of copolymer. If the content falls smaller than 0.1 mass %, the shortage will be at a disadvantage in unduly degrading the film-forming property of the composition. Conversely, if the content exceeds 50 mass %, the excess will be at a disadvantage in degrading the solubility and the dispersibility of the composition in a solvent, rendering formation of an uniform film difficult, and forming a cause for uneven application of the composition to a surface.

The alcohol solvent to be used herein is the same as the alcohol solvent which is used in the cosmetic coating composition mentioned above. The content of the alcohol solvent in the composition is in the range of 25-99 mass %, preferably 30-95 mass %. If the content falls smaller than 25 mass %, the shortage will be at a disadvantage in rendering solution of the polymer difficult. Conversely, if the content exceeds 99 mass %, the excess will be at a disadvantage in unduly lowering the content of the polymer and imparting insufficient gloss to the formed film.

The chromatic color type pigment does not need to be particularly discriminated. It may be the same as the powdered pigment which is used in the cosmetic coating composition as described above. When the chromatic color type pigment is mixed with a white color type powdered pigment such as titanium oxide, mica titanium or pearl pigment, the chromatic color can be generated in a pearly tone with added vividness. The white color type powdered pigment just mentioned may be the same as the powdered pigment described above with respect to the cosmetic coating composition. The content of the chromatic color type pigment in the composition does not need to be particularly discriminated. It is preferably in the range of 0.1-40 mass %, more preferably 1-20 mass %, based on the total mass of the composition. If the content falls smaller than 0.1 mass %, the shortage will be at a disadvantage in unduly lowering the effect of addition of the pigment. Conversely, if the content exceeds 40 mass %, the excess will be at a disadvantage in inducing uneven application of the composition.

Further, the composition is allowed to be added therein ethyl cellulose as a dispersant. The ethyl cellulose so added as a dispersant enables the pigment, for example, to be retained in a satisfactorily dispersed state for a long time and hardly entails precipitation or separation of a liquid layer as compared with other dispersants.

The chromatic color coating composition of this invention can further be added therein such additives as surfactant, bactericidal agent, caries preventing agent, dentin reinforcing agent, enzyme, edulcorant, antiseptic agent, flavoring agent, and stabilizing agent. These additives may be the same as those which are used in the cosmetic coating composition mentioned above.

The chromatic color coating composition of this invention can be produced by the method described above with respect to the cosmetic coating composition. The viscosity of this coating composition is preferred to be the same as that of the cosmetic coating composition mentioned above.

The chromatic color dental coating composition of this invention is capable of imparting heretofore unattainable fashioning property and decorating property to teeth by tinting the teeth not in a color natural thereto but in a chromatic color, such a pastel color of blue, pink, or light green, for example.

Now, the coating composition containing microfine leaves of this invention will be explained below. The coating composition containing microfine leaves is characterized by comprising a polymer selected from the group consisting of acrylic acid type copolymers, vinyl acetate type copolymers, and pyrrolidone type copolymers, ethyl cellulose, an alcohol solvent, and microfine leaves. By adding ethyl cellulose, it is made possible to attain uniform dispersion of microfine leaves which have a large specific gravity and are deficient in dispersion stability and retain the state of uniform dispersion for a long time.

The acrylic acid type copolymers and the vinyl acetate type copolymers are invariably usable herein on the condition that they possess a film-forming property. Specifically, they may be the same as those which are used in the chromatic color type coating composition mentioned above.

The alcohol solvent to be used herein may be the same as that which has been described above with respect to the cosmetic coating composition mentioned above. The content of the alcohol solvent in the composition is preferably in the range of 25-99 mass %, and more preferably in the range of 30-95 mass %, based on the total mass of the composition. If the content falls smaller than 25 mass %, the shortage will be at a disadvantage in rendering solution of the polymer difficult. Conversely, if the content exceeds 99 mass %, the excess will be at a disadvantage in unduly lowering the content of the polymer and imparting only insufficient gloss to the formed film.

The microfine leaves used herein may be the same as those used in the cosmetic coating composition mentioned above. The content of the microfine leaves in the composition does not need to be particularly discriminated. Generally, it is in the range of 0.1-40 mass %, preferably in the range of 1-20 mass %, based on the total mass of the composition. If the content falls smaller than 0.1 mass %, the shortage will be at a disadvantage in not attaining sufficient brilliance or coloration. Conversely, if the content exceeds 40 mass %, the excess will be at a disadvantage in unduly heightening viscosity and inducing uneven application of the composition.

Ethyl cellulose as used herein is effective in uniformly dispersing the microfine leaves in the composition by gelating a solution or imparting proper viscosity to the solution. It enables the microfine leaves immediately after being stirred to be dispersed in an extremely fine state and retained in the dispersed state for a long time as compared with other cellulose derivatives. When the composition is applied to teeth, therefore, it is enabled to form a coating film having the microfine leaves uniformly dispersed therein instead of being aggregated or unevenly distributed and consequently enhance the beauty and the brilliance of the appearance.

The content of ethyl cellulose in the composition is in the range of 0.1-30 mass %, preferably in the range of 1-15 mass %, based on the total mass of the composition. If the content falls smaller than 0.1 mass %, the shortage will be at a disadvantage in preventing the composition from acquiring thorough dispersion stability. Conversely, if the content exceeds 30 mass %, the excess will be at a disadvantage in unduly heightening the viscosity of the composition and suffering the composition to be applied unevenly.

Further, the coating composition containing the microfine leaves of this invention is allowed to be added therein such additives as surfactant, bactericidal agent, caries preventing agent, dentin reinforcing agent, enzyme, edulcorant, antiseptic agent, flavoring agent, and stabilizer. These additives are preferred to be the same as those which are used for the dental coating composition mentioned above.

The coating composition containing the microfine leaves of this invention can be produced in the same manner as the cosmetic coating composition mentioned above. The viscosity of the coating composition containing the microfine leaves is preferred to be equal to that of the dental coating composition mentioned above.

The dental coating composition containing the microfine leaves according to this invention is enabled to afford heretofore unattainable fashioning property and decorating property by adopting for the purpose of beautifying teeth such a decorative substance as lamé which has been used to date as a decorative material mainly for nails and face.

Now, the method for applying the cosmetic coating composition of this invention will be explained below. The cosmetic coating composition of this invention can produce the effect thereof amply by being applied in its unmodified form to nails or teeth. When the coating composition containing a chromatic color type pigment or microfine leaves is used, more beautiful coloration can be obtained by applying the coating composition containing a white color type pigment according to this invention as a primer to nails or teeth and applying a coating composition containing a chromatic color type pigment or microfine leaves as an overcoat thereon than when the coating composition containing a chromatic color type pigment or microfine leaves is applied by itself. The reason for this greater beautification is that the primer covering the inherent color of the surface of nails or teeth serves the purpose of enhancing and vivifying the coloration and the gloss of the overcoat. Moreover, since the primer covers the irregularities and scratches of the surface of teeth to a certain extent, the composition can be uniformly applied easily and the coating composition applied as an overcoat can bring an operational effect of exalting the quick-drying property.

Now, the remover of this invention will be explained below. The remover of this invention is characterized by gelling an alcohol solvent by mixing the alcohol solvent with a gelling agent. By this gelation, the remover is enabled to acquire fully satisfactory viscosity for the purpose of the removal aimed at. Further, the alcohol component which has been gelled allows the coating composition applied to teeth to be easily removed by the friction with an ordinary toothbrush.

Any alcohol solvent is usable as long as it is based on an alcohol. Preferably, it is a lower alcohol such as, for example, ethanol. The content of the alcohol solvent in the composition is not less than 20 mass %, preferably not less than 40 mass %, based on the total mass of the composition. If the content falls less than 20 mass %, the shortage will be at a disadvantage in unduly lowering the effect of removing the applied coating composition. The upper limit of the content is not particularly defined because it depends on the kind of a gelling agent to be used.

The gelling agent is effective in not only heightening the dispersing property of such additives as an abrasive but also wetting the alcohol component and consequently forming a gel with proper viscosity. Concrete examples of the gelling agent include, but not limited to, cellulose derivatives such as ethyl cellulose, hydroxyethyl cellulose, hydroxypropyl cellulose, and carboxymethyl cellulose, macromolecular polymers such as (meth)acrylic acid type copolymers, N-vinyl-2-pyrrolidone type copolymers, polyvinyl pyrrolidone, polyvinyl acetate, and vinyl acetate type copolymers, polysaccharides such as sodium alginate, dextran sodium sulfate, chondroitin sodium sulfate, pectins, carrageenin, galactomannan, and gum arabic, viscosity-enhancing silica, montmorillonite, and bentonite. These gelling agents may be used either singly or in the form of a combination of two or more members. Among other gelling agents enumerated above, hydroxypropyl cellulose or ethyl cellulose is particularly advantageous.

The content of the gelling agent in the composition is not particularly defined because it is varied with the kind of a substance for which it is used. In the case of ethyl cellulose, the content is in the range of 0.1-20 mass %; in the case of hydroxypropyl cellulose, in the range of 0.1-7.5 mass %, preferably 0.5-5 mass %; in the case of a t-butyl acrylate-ethyl acrylate-methacrylic acid copolymer, in the range of 1-40 mass %, preferably 3-20 mass %; in the case of N-vinyl-2-pyrrolidone-vinyl acetate copolymer, in the range of 0.2-30 mass %, preferably 1-15 mass %; and in the case of polyvinyl pyrrolidone, in the range of 0.1-20 mass %, preferably 0.5-10 mass %. If the content falls smaller than the each lower limit, the shortage will be at a disadvantage in unduly lowering the viscosity of the formed gel. Conversely, if the content exceeds the each upper limit, the excess will be at a disadvantage in unduly heightening the viscosity and rendering difficult the work of using the composition by brushing.

Further, the remover of this invention is allowed, as occasion demands, to be added therein, but not limited to, for example, abrasives such as calcium phosphate, calcium hydrogen phosphate, sodium hydrogen phosphate, silicon dioxide, aluminum oxide, aluminum hydroxide, calcium carbonate, and silicon anhydride; alkyl phosphate such as sodium lauryl sulfate and sodium lauryl phosphate; acyl amino acid salts such as acyl sodium glutamate and acyl sodium sodium sarcosine; surfactants such as sucrose fatty acid esters, sorbitan fatty acid esters, and polyoxyethylene fatty acid esters; moisturizing agents such as glycerin and sorbit; dentin reinforcing agents and caries preventing agents such as sodium monofluorophosphate, sodium fluoride, and hdyroxy apatite; edulcorants such as sodium saccharate and sorbit; vitamin preparations such as vitamin C and vitamin E, flavoring agents and perfume oils such as menthol, peppermint, spearmint, and natural substances; bactericidal agents such as benzalkonium chloride; antiseptic agents such as benzoic acid, p-hydroxybenzoic acid, and paraben; antibacterial agents; deodorants; and stabilizers which are generally contained in dentifrices.

Now, the method for producing the remover will be explained below. The remover of this invention is obtained by first mixing an alcohol solvent with a gelling agent, then adding an abrasive, a surfactant and other additives, and stirring the resultant mixture till they form a uniform blend.

Now, the intraoral lip supporter of this invention will be explained below. The intraoral lip supporter is intended to be used during the application of the dental coating composition to teeth for the purpose of keeping the teeth in an exposed state and aiding in applying the coating composition and drying the applied layer of the composition.

The first embodiment of the supporter of this invention is characterized by using a plastic material manifesting viscosity at least during use, enabling the lips to be opened by being mounted between the gums and the lips, and serving the purpose of fixing the lips to the gums while keeping the teeth in an exposed state. The plastic material mentioned herein means a material which is simply and easily molded at normal room temperature. Concrete examples of the plastic material include clay and dough of wheat flour, etc. The plastic material possesses viscosity. Besides a material which constantly possesses viscosity, it may include materials which possess no viscosity prior to use and acquires viscosity by absorbing water prior to use.

Preferably, the plastic material is a polysaccharide or a material formed mainly of a polysaccharide. The supporter which is formed of such a material, when set at a proper position in the mouth, increases viscosity by absorbing moisture from such as the saliva in the mouth, and fixes the upper and lower lips to the gums through adhesion. To be used effectively herein, the material is only required to be capable of inflating and gaining in viscosity with absorbed water. Concrete examples of such a plastic material, but not limited to, cereals such as corn, wheat, oats, rice, and potato, starch from rhizomes and legumes, cellulose derivatives such as galactomannan, hydroxyethyl cellulose, hydroxypropyl cellulose, and sodium caraboxymethyl cellulose, and sodium alginate, dextran sodium sulfate, chondroitin sodium sulfate, pentins, carrageenin, galactomannan, and gun arabic. These plastic materials may be used either singly or in the form of a combination of two or more members.

Besides the plastic material mentioned above, macromolecular polymers such as (meth)acrylic acid type copolymers, N-vinyl-2-pyrrolidone type copolymers, polyvinyl pyrrolidone, polyvinyl acetate, and vinyl acetate type copolymers, viscocity-enhancing silica, montmorillonite, and bentonite may be added to the supporter either singly or in the form of a combination of two or more members for the purpose of supplementing characteristic properties such as viscosity, swelling tendency, and strength.

Further, the supporter is allowed to added therein such additives as dentin reinforcing agent, caries preventing agent, edulcorant, vitamin, flavoring agent, antibacterial agent, bactericidal agent, stabilizer, and blood circulation accelerator. The concrete examples of such additives are the same as those used in the remover as described above.

Now, the method for producing the supporter will be explained below. In the case of a clayey plastic material, first a polysaccharide or a material formed mainly of a polysaccharide, when necessary, is milled into a powdery state. The powder is mixed with a suitable amount of water till a slurry mixture is formed. This mixture is extrusion molded and heated generally at a temperature in the range of 150-250°C for a duration in the range of 1-30 minutes. The proper heating conditions are not particularly defined because they are variable with the kind of material. By this heating, the water in the mixture is gasified into steam and the mixture is caused to form a spongy texture therein. The supporter of this invention is completed by drying the heated mixture. Since the supporter of this invention continues to possess plasticity, it can be freely molded even after it has been dried.

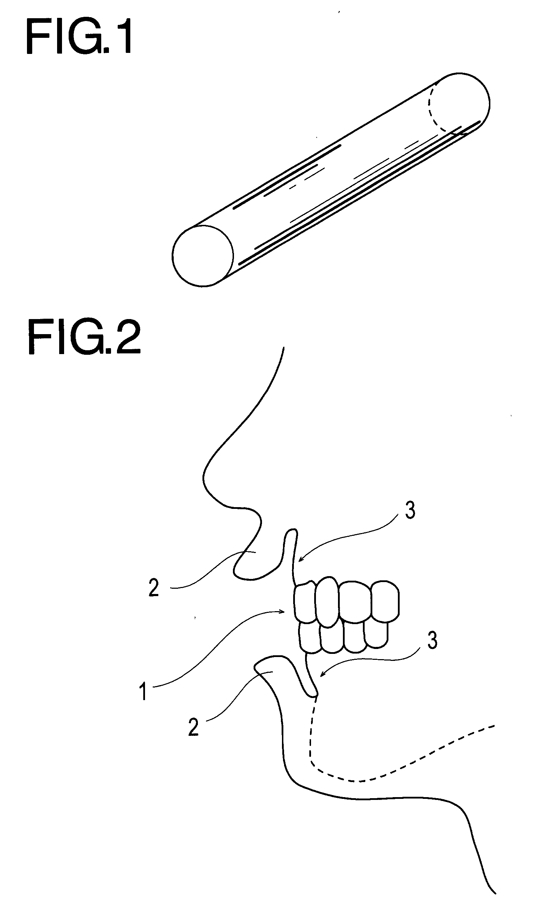

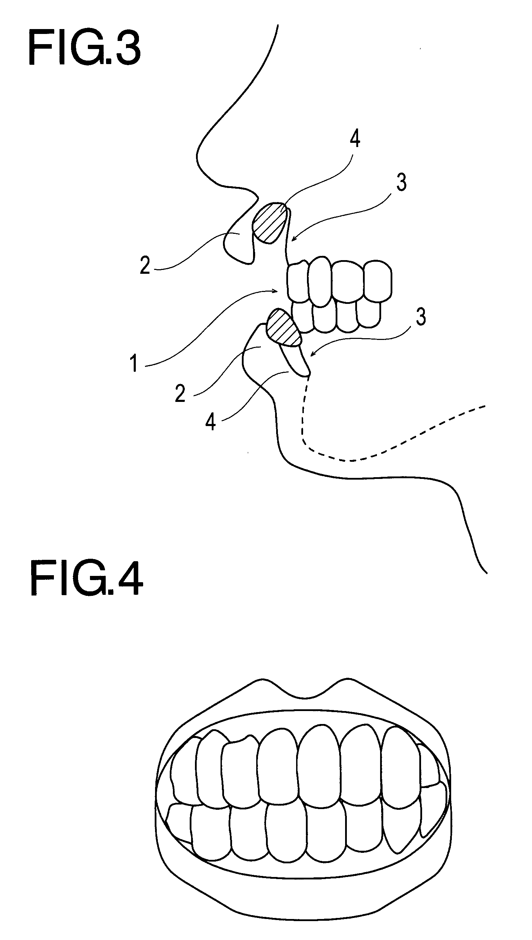

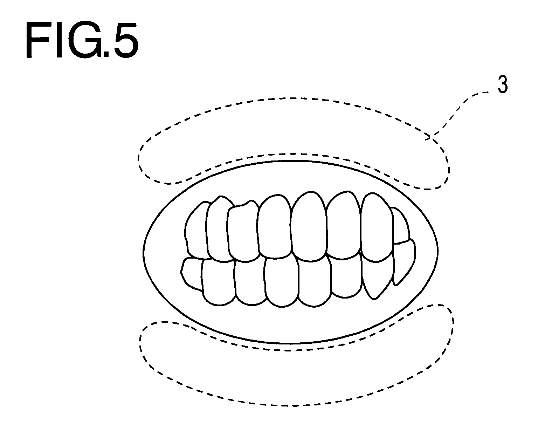

The method of using the supporter will be explained below with reference to the accompanying drawing. Fig. 1 is a diagram illustrating one example of the shape of the supporter of this invention prior to use, Fig. 2 is a cross section illustrating the state of the intraoral cavity having the lips opened in the vertical direction to expose the teeth before the supporter of this invention is set in position, Fig. 3 is a cross section illustrating the state of the intraoral cavity having the supporter of this invention set in position therein, Fig. 4 is a diagram illustrating the external appearance of the mouth having the supporter of this invention set in place therein, and Fig. 5 is a diagram which indicates the supporter with a broken line in the external appearance of Fig. 4 for the sake of facilitating comprehension of the state of attachment of the supporter of this invention. First, lips 2 are opened to expose teeth 1 in such a manner as to avoid touching the teeth. When a supporter 4 is set in position in front of gums 3 so as to fix the lips in the resultant state. Such a state as is illustrated in Fig. 3 is consequently assumed. Owing to the moisture in the mouth which originates in the saliva, for example, the supporter instantly acquires increased viscosity and enables the lips to be fixed in front of the gums and the teeth to be exposed through the parted lips. The work performed to this point can be immediately shifted to the work of applying the coating composition to the teeth. The supporter can be easily removed after use. When it partly retains in the mouth, it is totally harmless.

The second mode of embodying the supporter of this invention is characterized by possessing a structure curved in conformity with the shape of gums and serving the purpose of opening the lips by being mounted between the gums and the upper and lower lips while keeping the teeth in an exposed state. It will be explained below with reference to the accompanying drawings.

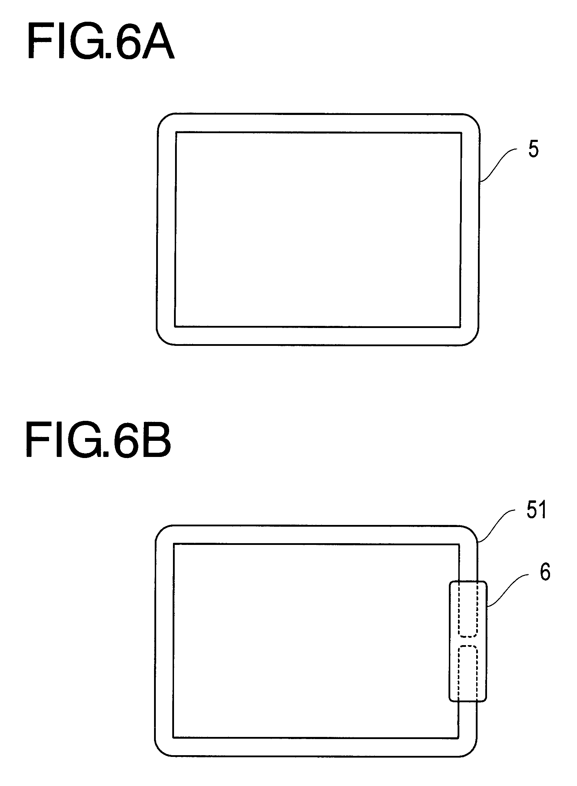

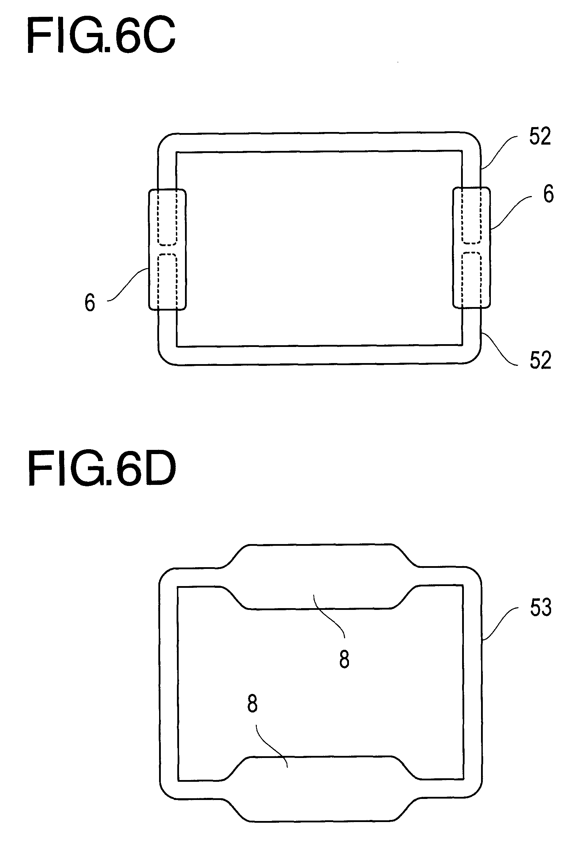

Fig. 6 is a front view of a lip supporter, depicting the mode of embodying the supporter of this invention. This supporter is formed of a lip supporter as illustrated in Fig. 6A and is only required to be in a tetragonal shape. Specifically, the lip supporter which is in an annular shape A, that which is in a shape opened in one part B, and that which is in a shape opened at two points C are conceivable modes of embodiment. Further, the lip supporter which is formed in a large width as illustrated in Fig. 6D may be used as a partly modified mode of embodiment.

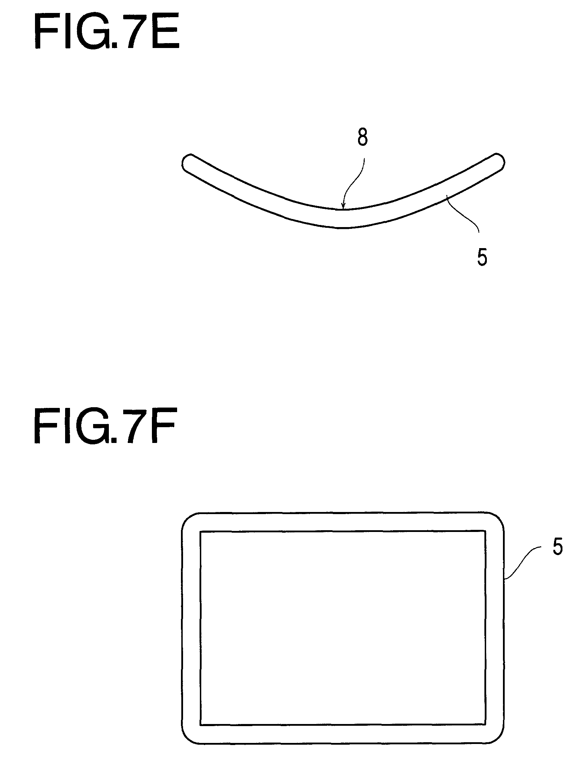

Fig. 7 is a plan view E and a front view F of the lip supporter. Sites 8 of an opened lips supporting member 5 which contact on the gums is bent for the purpose of heightening the closeness of contact.

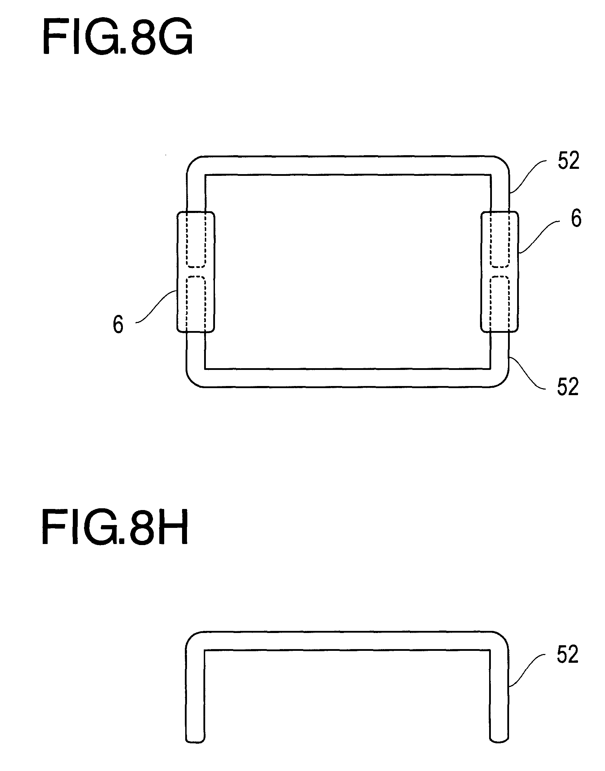

A preferred mode of embodiment will be explained below with reference to Fig. 8. The opened lips supporting member 5 may be included an example of the supporter having two members 52 each of the shape of three sides of square formed in a tetragonal shape with a connecting member 6. The member of the shape of three sides of square is formed as illustrated in Fig. 8H and is naturally bent as illustrated in Fig. 7E. It may be tapered off toward the leading end or notched for the purpose of facilitating the insertion thereof into the inserting mouth.

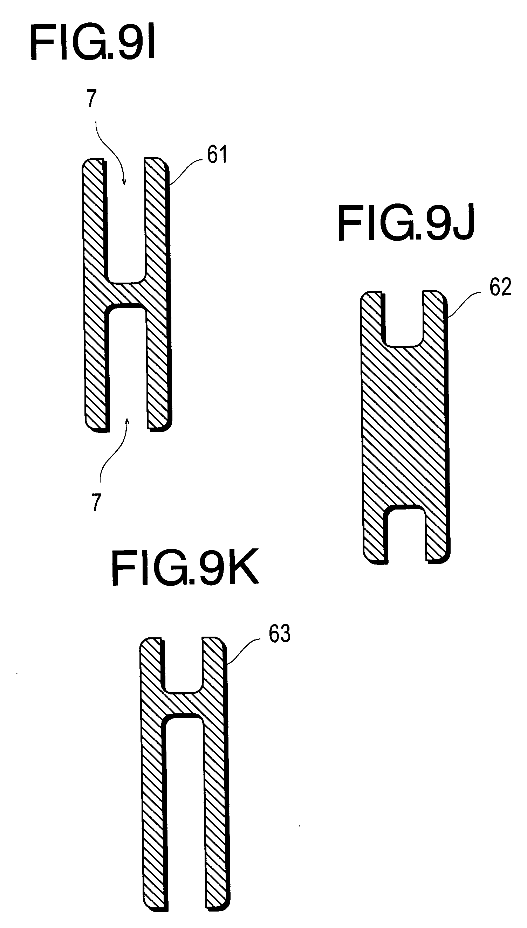

In this mode of embodiment, the width of the supporter may be adjusted by using connecting members 61-63 having respective inserting mouths 7 formed in different depths as illustrated in Fig. 9. The height of the supporter decreases by selecting a connecting member 61 having an insertion mouth 7 of a great depth and it increases by selecting a connecting member 62 having an insertion mouth 7 of a small depth. When a plurality of connecting members having inserting mouths of different depths are ready for use, the size of the supporter may be adjusted to suit the size of the user's mouth in an opened state by using the member of the shape of three sides of square of a fixed size. Besides, connecting members 63 having an upper and a lower inserting mouth 7 differing in depth may be used.

The opposite terminals of the member of the shape of three sides of square may be different in length in the lateral direction. By combining two members of the shape of three sides of square having lateral sides differing in length in a staggered pattern, the supporter may be formed in a tetragonal shape.

As another preferred mode of embodiment, the supporter having the part of an opened lips supporting member 53 destined to contact the gums formed in a large width as compared with the other part may be cited. In this case, since the part 8 destined to contact the gums is formed in a greater width, the area of contact between the opened lips supporting member and the gums can be enlarged and the pressure exerted on the gums can be dispersed, with the result that the comfort of the use of the supporter in the mouth will be increased. Owing to the absence of use of such a shock absorbing member as described above, the method of use and the cleaning can be attained simply.

Concrete examples of the material for the opened lips supporting member and the connecting member include, but not limited to, metals, alloys, and plastic substances. Among other materials mentioned above, plastic substances prove particularly advantageous.

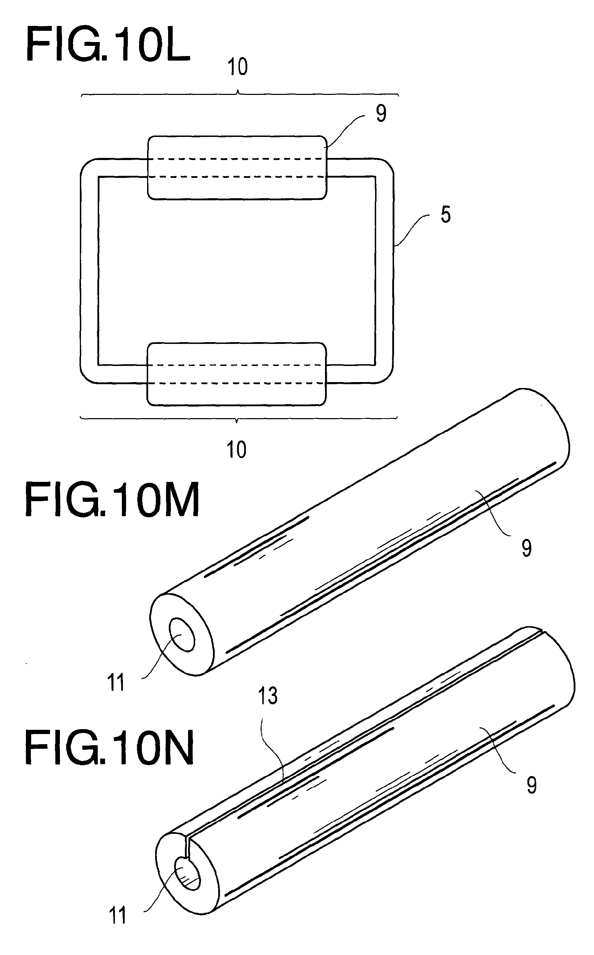

As yet another mode of embodiment, the supporter which further comprises a shock absorbing member 9 encircling a part 10 of the opened lips supporting member destined to contact the gums as illustrated in Fig. 10 L may be cited. The shock absorbing member specifically is a cylindrical member possessed of a hollow part 11 corresponding to the diameter of the opened lips supporting member as illustrated in Fig. 10M. It is furnished for the purpose of getting rid of such displeasure and pain as are caused by direct contact of the gums with the opened lips supporting member.

The shock absorbing member mentioned above is put to use by having the opened lips supporting member inserted through the hollow part 11. The mode having a notch 13 formed parallel to the opened lips supporting member as illustrated in Fig. 10N is also conceivable. Owing to this construction, the frame which is assembled in a tetragonal shape without forcing any breakdown therein can be easily attached and detached. The shock absorbing member (Fig. 10M) which is not furnished with a notch is put to use by inserting the opened terminal of the opened lips supporting member of Fig. 6B or 6C into the hollow part of the shock absorbing member and moving the shock absorbing member to a proper position. The present shock absorbing member excels hygienically in the sense that it can be easily cleaned after use.

The material for the shock absorbing member is preferred to possess elasticity. As concrete examples of suchmaterial, polyurethane, polyethylene, and silicone rubber may be cited, though not exclusively. Among other materials mentioned above, polyethylene proves particularly advantageous in the sense that it is capable of repelling water.

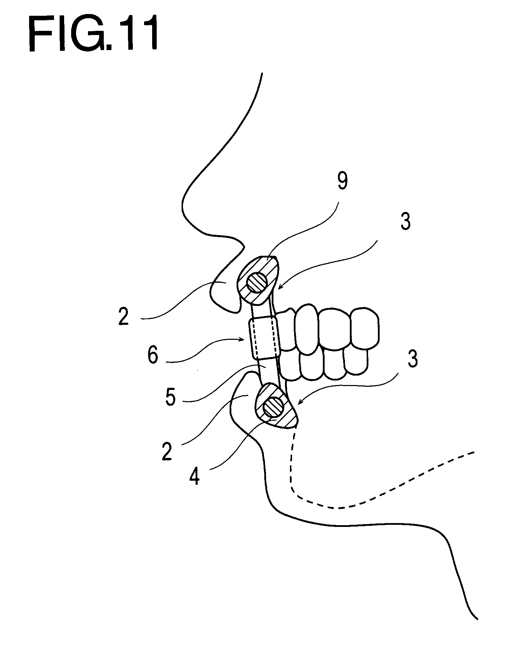

As one example of the method for using the supporter of this invention, the method for using the supporter illustrated in Fig. 10C will be explained below with reference to the drawings. Fig. 11 is a cross section illustrating the state of the intraoral cavity in which the supporter in the mode of embodiment furnished with a shock absorbing member is set in position for use. First, the frame is formed by selecting a connecting member 6 fit for the size of the user's mouth. Then, the lips 2 are opened and the supporter is mounted between the gums 3 and the lips 2 and the shock absorbing member 6 is attached tightly thereto. As a result, the lips 2 are fixed in the state illustrated in Fig. 11 so as to avoid touching the teeth. Then, this work of fixing can be shifted immediately to the work of applying the coating composition. The supporter of the other mode of embodiment is used in the basically same method as mentioned above.

### Examples

Now, this invention will be described more specifically below with reference to the examples. It should be noted, however, that this invention does not need to be limited to these examples.

In the following examples and comparative examples, Luvimer (made by BASF) which is a t-butyl acrylate ethyl acrylate methacrylic acid copolymer, Leoarl MS-100 (made by Lion Co.) which is an alkyl (meth)acrylate (meth)acrylic acid copolymer, and an isobutyl methacrylate N-octylacrylic acid amide acrylic acid copolymer were used ass the polymer I. The isobutyl methacrylate N-octylacrylic acid amide acrylic acid copolymer mentioned above was composed of isobutyl methacrylate : N-octylacrylic acid amide : acrylic acid at a molar ratio of 50 : 27 : 23.

PVP/VA E335 (a copolymer composed of N-vinyl-2-pyrrolidone (VP) and vinyl acetate (VA) at a molar ration, VP : VA, of 3 : 7) which is a N-vinyl-2-pyrrolidone vinyl acetate copolymer and E535 (a copolymer similarly composed of VP and VA at a molar ratio of 1 : 1) (both made by ISP Corp) were used as the polymer II; Luviskol K-30 (weight-average molecular weight: about 45,000) and K-90 (weight-averagemolecularweight: about 1,200,000) (bothmade by BASF) were used as the polyvinyl pyrrolidone; NZ-2 (degree of polymerization: 200), NZ-3 (degree of polymerization: 350), and NZ-5 (degree of polymerization: 500) (all made by Nihon Gosei Kagaku Kogyo Co.) were used as the polyvinyl acetate; and Ethyl cellulose N-200 (made by Hercules Co.) was used as the ethyl cellulose.

In the following examples and comparative examples, Flamenco Super Pearl(average particle diameter 25 *µ*m)(made by Inoue Koryo Co.), Flamenco Satin Pearl(average particle diameter 5 *µ*m), Flamenco Ultra Silk (average particle diameter 10 *µ*m), Flamenco Satina(average particle diameter 20 *µ*m), and Flamenco Velvet(average particle diameter 10 *µ*m)(all made by Ishihara Sangyo Co.) were used as the mica titanium. Then, CR50(made by Ishihara Sangyo Co.) was used as the titanium oxide and Epo Crystal 0.01(tetragon actually measuring 0.15 mm × 0.15 mm) and Epo Crystal(tetragon actually measuring 0.2 mm × 0.2 mm)(both made by Nakatsuka Kinzoku Hakufun Kogyo Co.), Prominence RYF(gold lamé)(made by Nihon Koken Kogyo Co.), Daiya Hologram S40EP(Daiya Kogyo Co.), and DC Glitter Gold 1#0.1(made by Dia Chemco Co.) were used as the lamé.

As respects the chromatic color pigment used in the following examples and comparative examples, Cloisonne Green was used as the green pigment, Cloisonne Violet (made by Inoue Koryo Co.) as the purple pigment, Colorona Imperial Red as the red pigment, and Colorona Dark Blue as the blue pigment (all made by Merck Japan Co.).

### <Example of Synthesis: Synthesis of acrylic acid type copolymer>

In a flask, 590 g of dimethoxy ethane, 13.0 g of itaconic acid, 185.7 g of t-butyl methacrylate, and 4.1 g of a monomer of the following chemical formula 3: 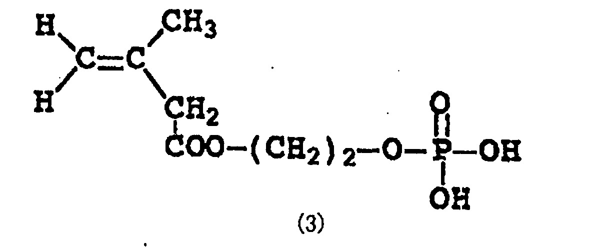 were placed, bubbled with argon gas fed at a flow volume of 1.5 L/min. and heated to an elevated temperature of 60°C. To the hot mixture in the flask, a solution of 1.703 g of 2,2-azobis(2,4-dimethylvaleronitrile) in 51 g of dimethoxyethane was added dropwise over a period of 30 minutes. After this addition, the reactants were left reacting at 60°C for four hours and at 75°C for four hours. The resultant reaction mixture was thrown into 18 liters of an ethanol : water = 2 : 1 (mass ratio) solution, purified therein twice, and then dried with hot air at 60°C overnight to obtain a copolymer (hereinafter referred to as copolymer A) (yield 100 g).

### <Example 1: Preparation of coating composition>

A coating composition of this invention was obtained by mixing 21 g of Luvimer 100P, 10 g of PVA/WA E335, 5 g of Flamenco Super Pearl and 64 g of ethanol and stirring them together by the use of a homomixer at 5000 rpm for five minutes.

### <Examples 2-10: Preparation of coating compositions>

Coating compositions shown in Table 1 were prepared by following the procedure of Example 1.

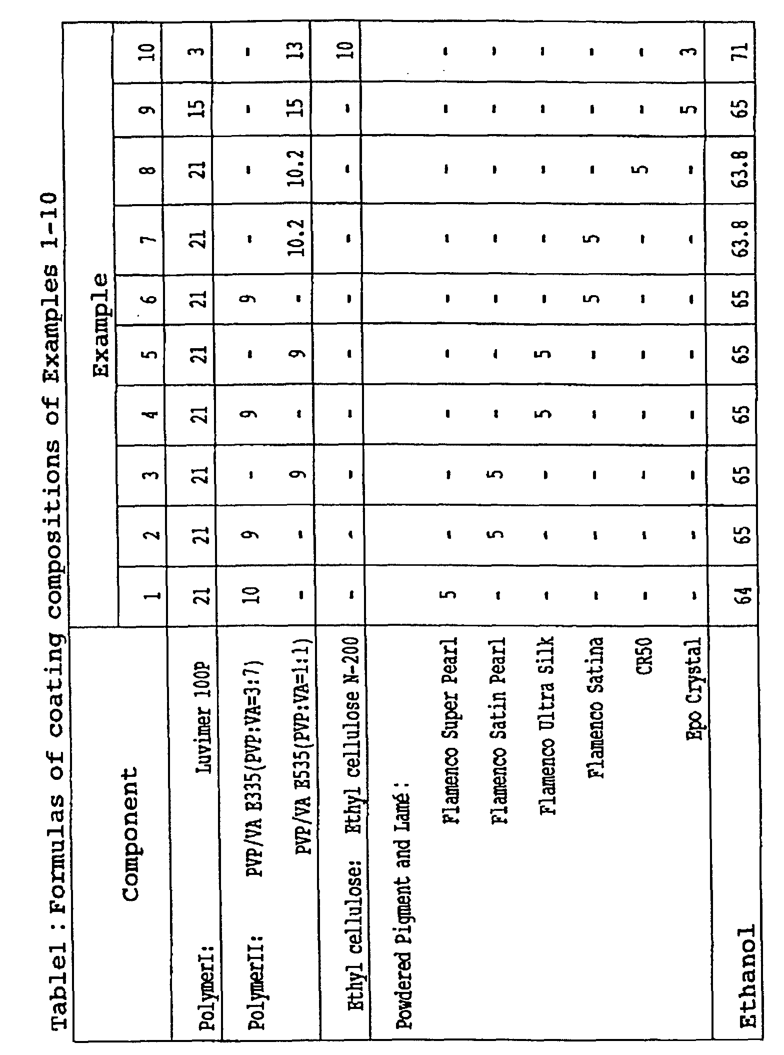

### <Comparative Examples 1-8: Preparation of coating compositions>

The coating compositions of the comparative examples shown in Table 2 were prepared by following the procedure of Example 1. The compositions in these comparative examples were as shown below.
Comparative Example 1: Composition using polymer I of this invention
Comparative Example 2: Composition of Comparative Example 1 plus surfactant
Comparative Examples 3 and 4: Compositions using polyvinyl pyrrolidone in the place of polymer II of this invention
Comparative Example 5, 6, and 7: Compositions using polyvinyl acetate in the place of polymer II of this invention
Comparative Example 8: Composition using copolymer A as the base polymer

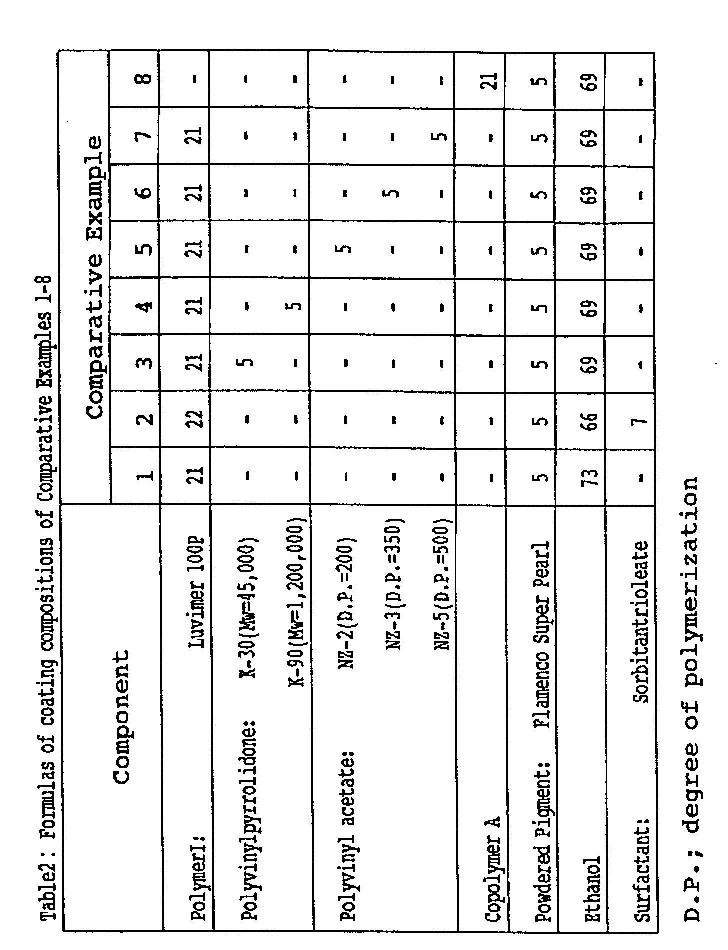

### <Test of compositions of Examples 2-10 and Comparative Examples 1-8 for physical properties and organoleptic examination thereof>

The coating compositions obtained in the examples and comparative examples mentioned above were tested for characteristic properties by the methods shown below.

### (1) Dispersion stability

The powdered pigment contained in the composition of each of the examples was tested for initial dispersibility immidiately after stirring and for dispersion stability after the following seven days' standing. The results were rated on the four-point scale, wherein ⓞ stands for total absence of precipitation or separation of liquid layer, ○ for slight occurrence of such phenomenon, Δ for positive occurrence of such phenomenon, and × for copious occurrence of such phenomenon.

### (2) Uneven application

A given coating composition was applied to bovine teeth and the applied layer was visually observed to determine whether it showed sign of uneven application.

### (3) Quick drying property

A given coating composition was applied to bovine teeth and the applied layer was touched with a finger tip at fixed intervals till dryness was confirmed by the fact that adhesion of a finger print to the film ceased to exist and the finger tip could be slid on the surface of the film (viscous dryness).

### (4) Gloss (luster)

Bovine teeth covered with a given coating composition were placed 2 m directly below a fluorescent lamp of 40 W and examined for gloss with unaided eyes.

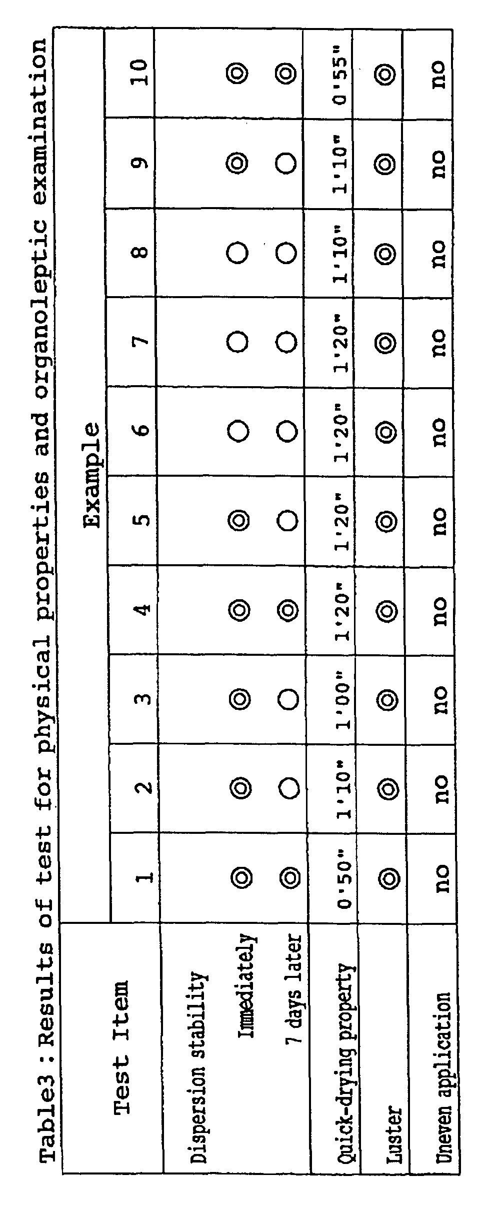

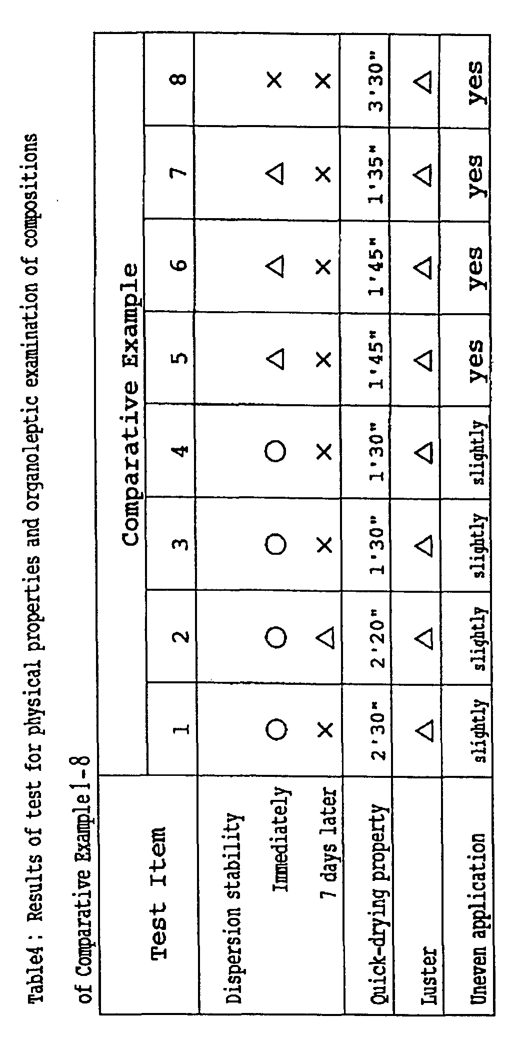

It is noted from Table 3 that the coating compositions of Examples 1-10 invariably showed satisfactory dispersion stability and retained this state of stability for close to one month, though more or less varied with the kind of pigment. The coating composition of Example 10 incorporating ethyl cellulose therein showed better dispersion stability than the coating compositions of Examples 9 and 10 incorporating therein lamé. In all the examples, addition of a surfactant resulted in exalting dispersion stability. It is noted from Table 4 that the coating compositions of Comparative Examples 1 and 2 which contained polymer I alone and those of Comparative Examples 3 and 4 which contained polyvinyl pyrrolidone as the polymer I invariably showed fair initial dispersibility but generated precipitation after the elapse of three days thereafter. The coating compositions of Comparative Examples 5-7 which contained polyvinyl acetate as the polymer I were invariably deficient in initial dispersibility. The coating composition of Comparative Example 8 produced an applied coat of inferior luster and the composition itself showed discernible precipitation and separation of liquid layer after one day's standing and was very deficient in dispersion stability.

Then, as regards the quick drying property, the coating compositions of Examples 1-10 reached the state of viscous dryness within periods in the range of 50 seconds-one minute 20 seconds. In contrast, those of comparative examples could not compare favorably with those of the examples of this invention, excepting that those of Comparative Examples 3 and 4 which contained polyvinyl pyrrolidone dried rather quickly among others of the comparative examples.

As regards the uneven application, the coating compositions of Examples 1-10 produced satisfactory results as shown in Table 3. In contrast, of the coating compositions of the comparative examples shown in Table 4, those of Comparative Examples 5-7 which showed poor dispersion stability immediately after stirring induced conspicuous signs of uneven application. It is inferred that the luster was affected because the uneven application rendered formation of a uniform film difficult.

The bovine teeth to which the coating compositions of Examples 1-10 were applied were tested for resistance to pollution. Specifically, when the coated bovine teeth were smeared with such foodstuffs as various flavoring materials and perilla leaves and then left standing for one hour, they were not polluted with the colors of the foodstuffs because the foodstuffs could be easily washed off with water. The coated bovine teeth were tested for physical resistance. Specifically, when a block of sponge impregnated with 18% (v/v) ethanol was reciprocally rubbed on the teeth up to more than 100 repetitions, they should no sign of peeling of the coating film. Further, the coating compositions of Examples 9 and 10, when tested for the physical durability mentioned above, showed no sign of peeling of lamé.

When the bovine teeth to which the coating composition of Example 1 was applied were brushed with an ordinary dentifrice containing an abrasive by way of test for physical durability, they showed no sign of peeling of the applied layer even after more than 200 brushing motions. This means that this coating composition enables its user to enjoy continued beautification of his teeth without changing his everyday custom such as brushing.

From the results reported above, it is noted that the addition of a pyrrolidone type copolymer to an acrylic acid type copolymer results in imparting veritably outstanding characteristic properties as compared with the addition of polyvinyl pyrrolidone or polyvinyl acetate by itself.

### <Examples 11-15: Coating compositions containing ethyl cellulose and lamé>

Coating compositions containing ethyl cellulose and lamé as shown in Table 5 were prepared by following the procedure of Example 1.

### <Comparative Examples 9-13: Coating compositions containing other dispersants and lamé>

Coating compositions containing lamé shown in Table 5 were prepared by following the procedure of Example 1 while using hydroxypropyl cellulose (HPC), sodium carboxymethyl cellulose (CMC-Na), and hydroxyethyl cellulose (HEC) severally in the place of ethyl cellulose. As shown in Table 5, the contents of dispersant in these coating compositions were smaller than the contents of ethyl cellulose in the coating compositions of Examples 11-15. These smaller contents may be logically explained by a supposition that the coating compositions had lower degrees of solubility in an alcohol base and, when the amounts thereof exceeded those indicated in Table 5, they were not thoroughly dispersed. Thus, the coating compositions were prepared only with difficulty.

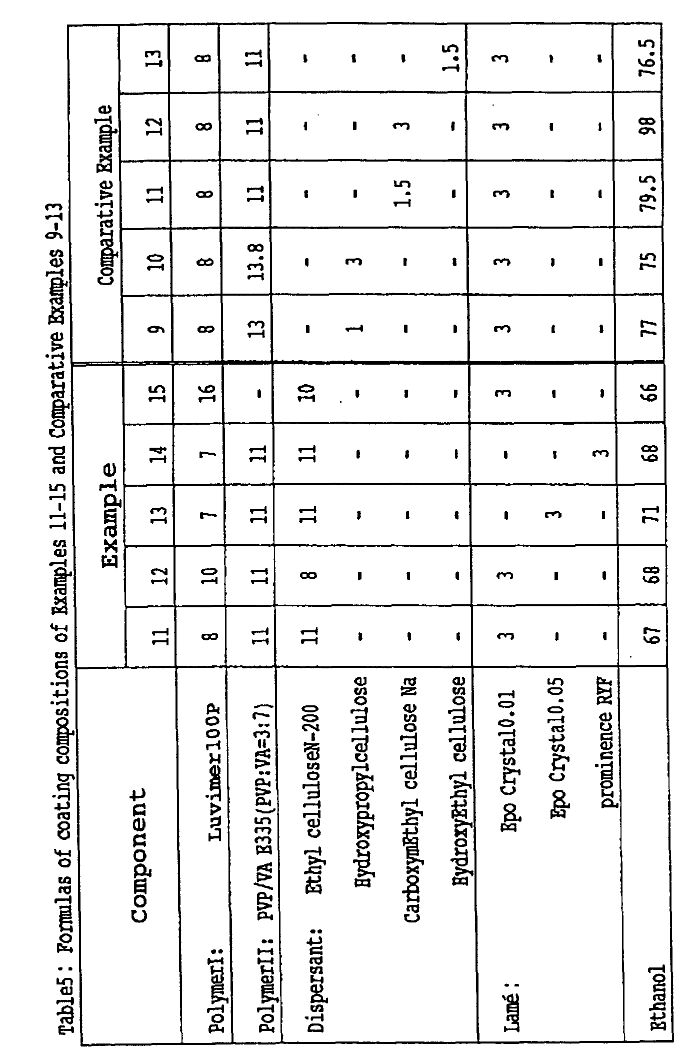

### <Dispersion Stability Test of coatinq compositions of Examples 11-15 and Comparative Examples 9-13>

The coating compositions of Examples 11-15 and Comparative Examples 9-13 were tested for dispersion stability. The method for performing the test was the same as the test of the coating compositions of Example 1-10 for physical properties. The results are shown in Table 6.

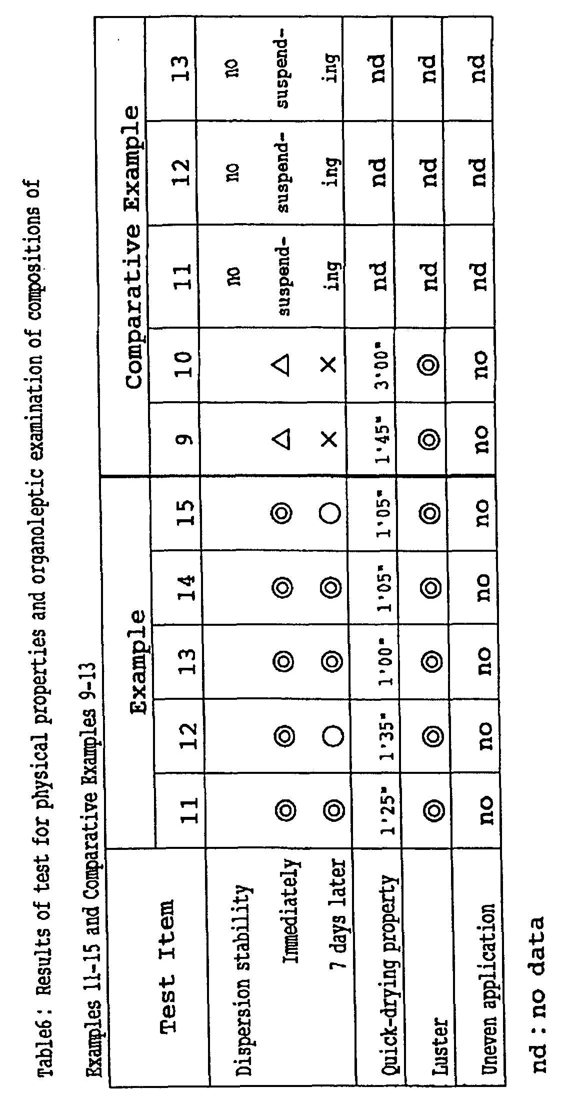

From the results reported in Table 6, it is noted that the coating compositions of Examples 11-15 invariably showed satisfactory initial dispersibility of lamé, showed nearly as good dispersibility after seven days' standing as immediately after stirring, and showed virtually no sign of precipitation of lamé or pigment or of separation of liquid layer. These coating compositions were found to excel not only in dispersion stability but also in film-forming property as evinced by the absence of any rise of viscosity or any decline of quick-drying property in consequence of the addition to the dispersion stability.

### <Examples 16-23: Chromatic color coating compositions containing ethyl cellulose>

According to the formulas of Table 7, coating compositions including ethyl cellulose and chromatic pigment were prepared by following the procedure of Example 1.

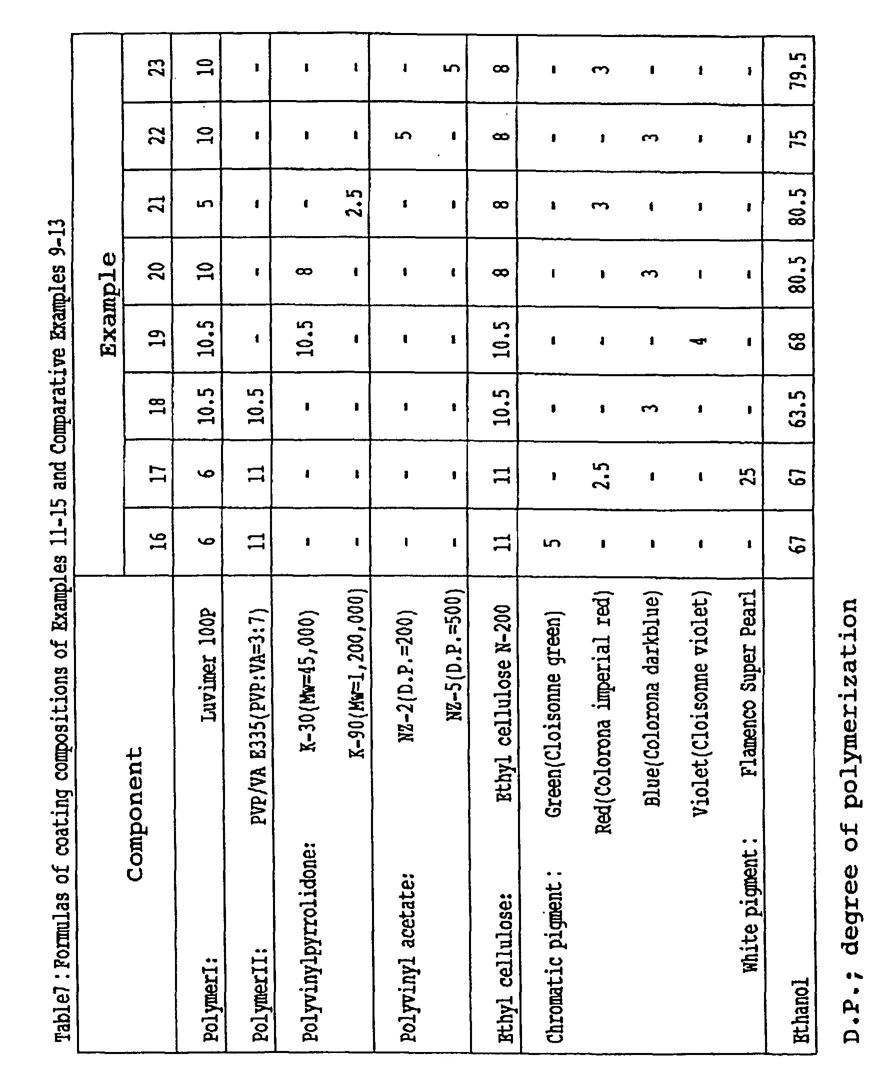

### <Dispersion Stability Test of the coatinq compositions of Examples 16-23>

The coating compositions of Example 16-23 were tested for dispersion stability. The method for performing this test was the same as that for the test of coating compositions of Examples 1-10 for physical properties. The results are shown in Table 8.

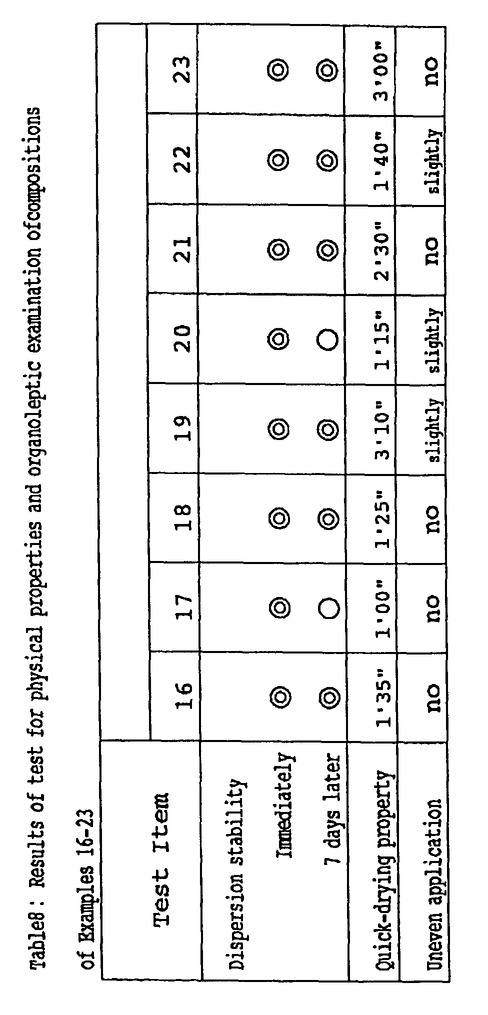

From the results reported in Table 8, it is noted that the coating compositions of Examples 16-23 invariably showed satisfactory initial dispersibility of pigment, showednearly as good dispersibility after seven days' standing as immediately after stirring, and showed virtually no sign of precipitation of pigment or of separation of liquid layer. These coating compositions were found to excel not only in dispersion stability but also in film-forming property as evinced by the absence of any rise of viscosity or any decline of quick-drying property in consequence of the addition to the dispersion stability. Further the coating compositions of Examples 15-23 invariably realized beautiful coloration and particularly the coating composition having Flamenco Super Pearl mixed with Colorona Imperial Red could form a pearly coating film possessed of a vivid pink color.

### <Examples 24-31: Coating compositions having several species of polymer I blended therein>

The coating compositions of the preceding examples contained Luvimer 100P solely as the polymer I. In the present examples, coating compositions further incorporating therein as the polymer I an alkyl acrylate N-octylacrylic acid amide copolymer, i.e. a copolymer containing N-octylacrylic acid amide as a monomer (hereinafter referred to as "copolymer B") or Leoarl MS-100 (made by Lion Co.), i.e. an alkyl (meth)acrylate (meth)acrylic acid copolymer were prepared and were examined to determine the effect of blending several species of polymer I.

The coating compositions containing the copolymer B and Leoarl MS-100 as shown in Table 9 were prepared by following the procedure of Example 1. Here, Examples 24-29 used the blend of three species of polymer I, i.e. Luvimer 100P, copolymer B, and Leoarl MS-100 and Examples 30 and 31 used the blend of two of these three species of polymer I.

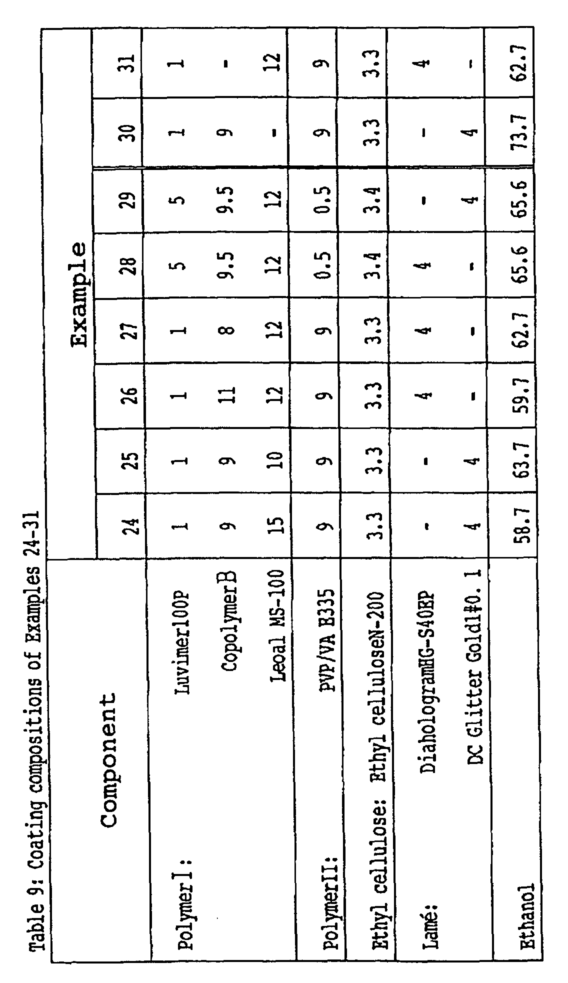

### <Dispersion Stability Test of the coating compositions of Examples 24-31>

The coating compositions of Examples 24-31 were tested for dispersion stability, quick-drying property, uneven application, and luster. The method for performing the test was the same as that of the test for physical properties performed in Examples 1-10. The results are shown in Table 10.

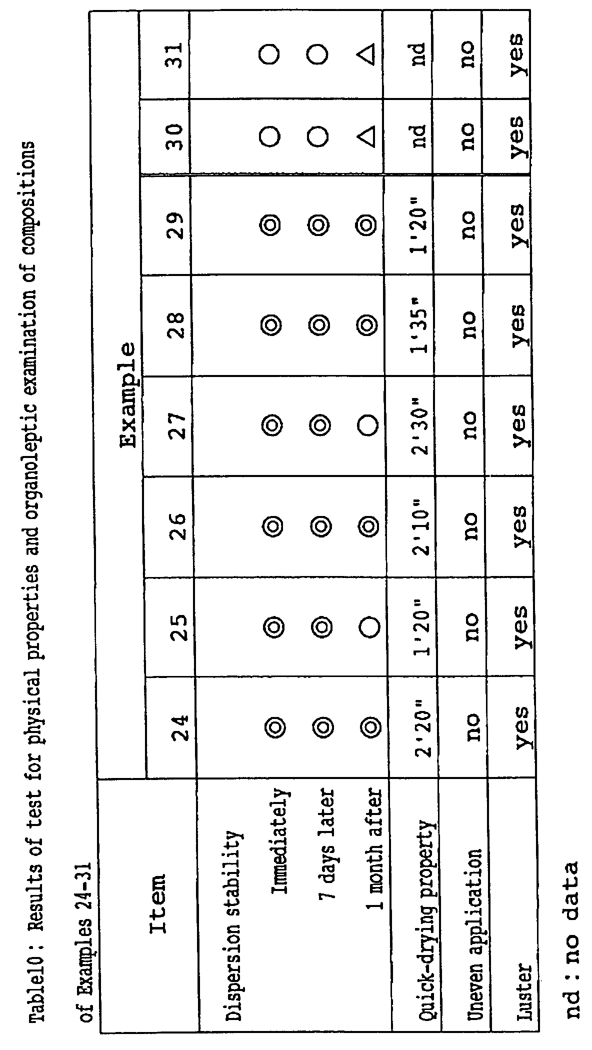

It is noted from Table 10 that the coating compositions of these examples invariably had large specific gravities, satisfactorily dispersed lamé particles measuring not less than 0.4 mm in width, and showed no conspicuous precipitation of lamb even after one week's standing. The coating compositions of Examples 24-29 incorporating three species of polymer therein showed particularly satisfactory dispersibility even after one month's standing as compared with the coating compositions of Examples 30 and 31 incorporating two species of polymer in a mixed state therein . Especially, the coating compositions of Examples 24 and 25 showed nearly as good dispersibility as immediately after dispersion. From these results, it is inferred that the mixing of Leoarl MS-100 resulted in conspicuously improvement of the dispersion stability of lamé.

Further, the coating compositions were tested for physical durability to determine the degree of peeling of lamb from the coating film. The test was performed by applying coating compositions of Examples 26 and 27 and Example 31 to the teeth of human subjects, allowing the subjects to take their daily meals (including consumption of alcoholic beverage), and making a visual observation of the coating films on their teeth periodically to determine the degree of peeling of the film. As a result, the coating composition of Example 17 (containing 8 mass % of copolymer B) withstood three meals and the coating composition of Example 26 (containing 11 mass % of copolymer B) could retain a satisfactory state without entailing peeling of not more than one third of the lamé even after three or more meals, whereas the coating composition of Comparative Example 31 (containing no copolymer B) suffered from peeling of not less than one third of the lamb in consequence of one meal. From these results, it is safely concluded that the adhesiveness of the lamb to the teeth could be improved by the mixing of a copolymer containing N-octylacrylic acid amide as a monomer therein.

### <Example 32: Effect of primer>

First, the coating composition of Example 1 mentioned above was applied to bovine teeth and the formed layer was labeled as a primer. Then, the coating compositions of Examples 10 and 11 (containing lamé) and Example 16 (chromatic color type) were applied thereto as top coats. The coating films thus formed and the other type of coating films obtained by directly applying top coats solely to bovine teeth while omitting application of a primer were visually observed to determine coloration.

As a result, the coating films using a primer were found to produce beautiful coloration of pigment and emit fine luster. Further, they subdued irregularities and scratches on the surface of teeth and realized a beautiful external appearance as compared with the coating films using no primer.

### <Example 33: Preparation of remover>

Forty (40) g of ethanol was weighed out and 2 g of hydroxypropyl cellulose was dissolved as a gelling agent therein. Then, 2 g of sodium lauryl sulfate, 0.1 g of sodium saccharate, and 7.5 g of glycerin were sequentially dissolved therein in the order mentioned and 48.4 g of sodium hydrogen phosphate was further mixed therewith. They were thoroughly stirred together to prepare a remover according to this invention.

### <Examples 34-37: Preparation of removers>

Removers of this invention having compositions shown in Table 11 were prepared by following the procedure of Example 33. The remover obtained in Example 37 used t-butyl acrylate/ethyl acrylate/methacrylic acid copolymer (Luvimer 100P) as the gelling agent.

### <Comparative Examples 14 and 15: Preparation of removers>

A remover of Comparative Example 15 having a composition shown in Table 11 was prepared by following the procedure of Example 33. Here, Comparative Example 15 represents the case of using a lowered ethanol concentration in the remover of this invention. Then, Comparative Example 14 represents the case of using a commercially available dentifrice.

### <Test of remover for characteristic properties>

The removers of the examples and the comparative examples mentioned above were taken each in a fixed amount of 3 g on a toothbrush. The toothbrushes were rubbed against the bovine teeth on which the coating composition of Example 1 had been applied and then dried. By counting one reciprocation of the toothbrush as one round, the number of rounds which preceded thorough peeling of the coating composition from the brushed part of teeth was determined.

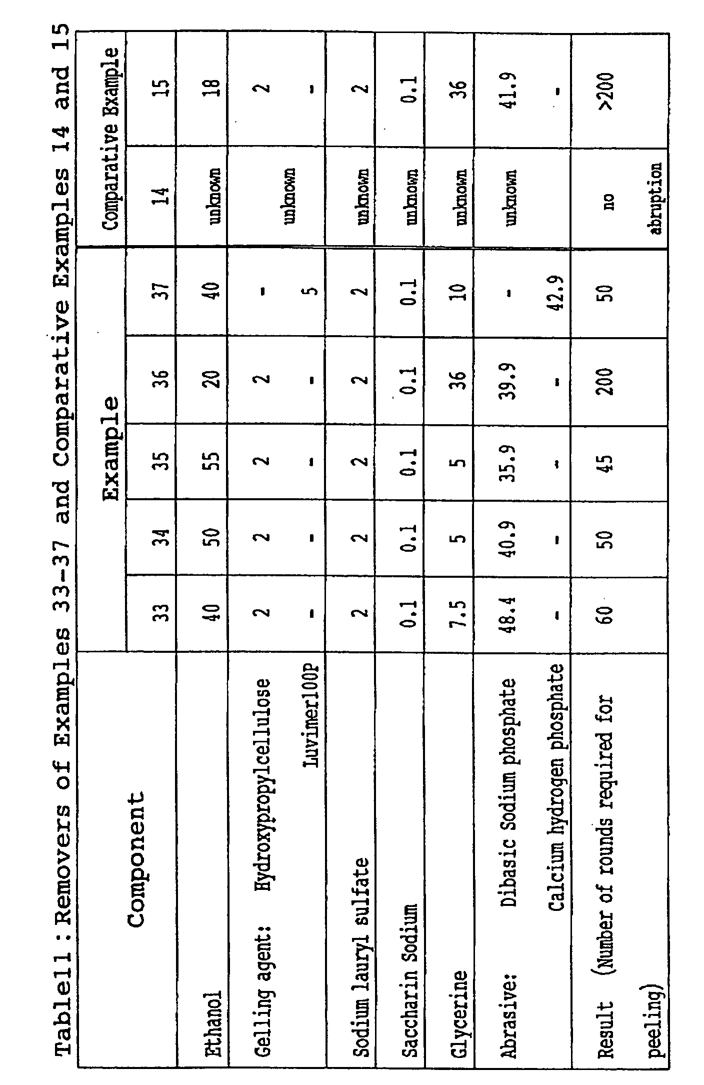

It is clear from Table 11 that the coating compositions of Examples 33-37 required small numbers of rounds of brushing for thorough removal thereof, whereas the coating composition of Comparative Example 15 required not less than 200 rounds of brushing because of a small alcohol content. The commercially available dentifrice of Comparative Example 14 defied peeling by not less than 200 rounds of brushing. This fact implies that the coating composition applied to the teeth could withstand the impact of ordinary toothbrushing.

### <Example 38: Intraoral lip supporter>

A supporter of this invention was obtained by adopting Ecofoam made by Oji Seitai Co.) as the material for the supporter and forming this material in the shape of a slender cylinder 15 mm in diameter and 45 mm in length. This supporter was mounted between lips and gums in accordance with the method described above and was kept under observation to examine the development.

As a result, the supporter acquired increased viscosity by absorbing the moisture such as of saliva in a matter of about one to three seconds and could fix the lips to the gums. Further, this supporter could retain such viscous force for a duration in the approximate range of 5-10 minutes and enable the coating composition of this invention to be applied to teeth and completely dried. It could be easily removed after use and, during the removal, would not inflict any injury to the mucous membrane in the oral cavity.

### <Example 39: Preparation of cosmetic coating composition>

The same compositions as those of Examples 11-15 and 16-23 mentioned above were prepared and processed to produce cosmetic coating compositions for application to the skin.

The entire disclosure of Japanese Patent Application No. 2000-121835 filed on April 21, 2000 and No. 2001-072640 filed on March 14, 2001 including specification, claims and summary are incorporated therein by reference in its entirely.

## Claims

1. A cosmetic coating composition comprising
at least one polymer I which is a copolymer of at least two monomers selected from the group consisting of monomers represented by the chemical formula 1: 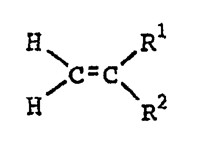 wherein, R¹ represents a hydrogen atom or a methyl group, R² represents -COOR³ or -CONHR³, wherein R³ represents a hydrogen atom or a linear, branched, or cyclic alkyl group of 1-10 carbon atoms,
at least one polymer II which is a copolymer of at least two monomers selected from the group consisting of monomers represented by the chemical formula 2: 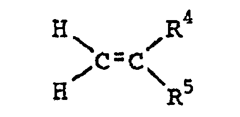 wherein, R⁴ represents a hydrogen atom or a methyl group, R⁵ represents -COOR⁶, an acetoxyl group, or a 2-pyrrolidyl group, wherein R⁶ represents a linear, branched, or cyclic alkyl group of 1-6 carbon atoms, providing that in at least one of said at least two monomers, R⁶ represents a 2-pyrrolidyl group), and
an alcohol solvent.

2. The composition according to claim 1, wherein a copolymer formed of at least two monomers selected from the group consisting of alkyl (meth)acrylates and (meth)acrylic acid is contained as said polymer I.

3. The composition according to claim 2, wherein said polymer I is a t-butyl acrylate-ethyl acrylate-methacrylic acid copolymer.

4. The composition according to any of claims 1-3, wherein said polymer I contains N-octylacrylic acid amide as the monomer of the chemical formula 1.

5. The composition according to claim 4, wherein said polymer I is an isobutyl methacrylate-N-octylacrylic acid amide-acrylic acid copolymer.

6. The composition according to any of claims 1-5, wherein a N-vinyl-2-pyrrolidone-vinyl acetate copolymer is contained as said polymer II.

7. The composition according to any of claims 1-6, which further comprises a powdered pigment.

8. The composition acording to claim 7, **characterized by** the fact that said powdered pigment is a chromatic color type pigment and applying on teeth.

9. The composition according to any of claims 1-8, which further comprises microfine leaves.

10. The composition according to any of claims 1-9, which further comprises ethyl cellulose.

11. A dental coating composition comprising at least one polymer selected from the group consisting of acrylic acid type copolymers, vinyl acetate type copolymers, and pyrrolidone type copolymers, an alcohol solvent, and a chromatic color type pigment.

12. The composition according to claim 11, which further comprises ethyl cellulose.

13. A cosmetic coating composition comprising at least one polymer selected from the group consisting of acrylic acid type copolymers, vinyl acetate type copolymers, and pyrrolidone type copolymers, an alcohol solvent, ethyl cellulose, and microfine leaves.

14. A remover for a dental coating composition, **characterized by** comprising an alcohol solvent and a gelling agent and containing said alcohol solvent in an amount of not less than 20 mass % based on the total mass of the remover.

15. The remover according to claim 14, which possesses viscosity in the range of 100-500 mPa · s.

16. The remover according to claim 14 or claim 15, wherein said gelling agent is a cellulose derivative, a (meth)acrylic acid type copolymer, polyvinyl acetate, polyvinyl pyrrolidone, or a N-vinyl-2-pyrrolidone type copolymer.

17. A remover according to claim 16, wherein said cellulose derivative is hydroxypropyl cellulose or ethyl cellulose.

18. An intraoral lip supporter for use in applying a dental coating composition and drying the applied dental coating composition, **characterized by** forming a plastic material possessing viscosity at least during the time of use and serving the purpose of wedging itself between gums and lips thereby opening the lips, and fixing the lips to the gums with the teeth kept meanwhile in an exposed state.

19. A supporter according to claim 18, wherein said plastic material contains a polysaccharide.

20. An intraoral lip supporter for use in applying a dental coating composition and drying the applied dental coating composition, **characterized by** possessing a construction curved in conformity with the shape of a gum and serving the purpose of wedging itself between the gums and the upper and lower lips thereby fixing the lips to the gums with the teeth kept meanwhile in an exposed state.

21. The supporter according to claim 20, which is formed of tetragonal opened lips supporting members(5).

22. The supporter according to claim 21, wherein the part of said opened lips supporting member not in contact with the gum is in an opened state and is connected with a connecting member(6).

23. The supporter according to claim 22, wherein said opened lips supporting member is formed by joining two members(52) each of the shape of three sides of a square in a tetragonal shape with connecting members(6).

24. A supporter according to any of claims 20-23, wherein said opened lips supporting member is so formed that the part thereof in contact with the gum has a greater width than the part thereof not in contact with the gum.

25. A supporter according to claim 23, wherein said width is adjusted by the use of said connecting member having an inserting mouth varying in depth.

26. A supporter according to any of claims 20-23 and 25, which further comprises a shock absorbing member(9) adapted to encircle the part of said opened lips supporting member in contact with the gum.

27. A supporter according to claim 26, wherein said shock absorbing member is formed of an elastic material.

28. A supporter according to claim 27, wherein said elastic material is polyethylene.

29. A method for applying a coating composition to nail or tooth, **characterized by** applying a composition set forth in any of claims 1-10 containing a white powdered pigment to the nail or tooth as a primer coating, and then overcoating a composition set forth in any of claims 1-13 containing a chromatic color type pigment or microfine leaves.

30. A method for applying a composition set forth in any of claims 1-13 to a tooth by the use of a supporter set forth in any of claims 18-28.
